(19)

Europäisches
Patentamt
European
Patent Office
Office européen
des brevets

(11) **EP 2 907 872 B1**

(12) **EUROPEAN PATENT SPECIFICATION**

(45) Date of publication and mention
of the grant of the patent:
**20.12.2017 Bulletin 2017/51**

(21) Application number: **13846152.0**

(22) Date of filing: **09.10.2013**

(51) Int Cl.:
*C12Q 1/68* [(2006.01)]    *C12N 15/10* [(2006.01)]
*C07H 21/04* [(2006.01)]    *A61K 31/711* [(2006.01)]

(86) International application number:
**PCT/RU2013/000892**

(87) International publication number:
**WO 2014/058355 (17.04.2014 Gazette 2014/16)**

(54) **METHODS AND COMPOSITIONS FOR THE QUANTITIVE ANALYSIS OF TERMINAL NUCLEOTIDES OF A G CHAIN OF HUMAN TELOMERIC DNA**

VERFAHREN UND ZUSAMMENSETZUNGEN ZUR QUANTITATIVEN ANALYSE VON TERMINALEN NUKLEOTIDEN EINER G-KETTE VON HUMANER TELOMER-DNA

PROCÉDÉS ET COMPOSITIONS POUR L'ANALYSE QUANTITATIVE DES NUCLÉOTIDES TERMINAUX DU BRIN G DE L'ADN TÉLOMÉRIQUE DE L'HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.10.2012 RU 2012143263**

(43) Date of publication of application:
**19.08.2015 Bulletin 2015/34**

(73) Proprietor: **Chiryasova, Elena Andreyevna
Leninsky raion, Moskovskaya oblast 142714 (RU)**

(72) Inventor: **Chiryasova, Elena Andreyevna
Leninsky raion, Moskovskaya oblast 142714 (RU)**

(74) Representative: **Spengler, Robert
Potthast & Spengler Patentanwälte
Küfergasse 11
89073 Ulm (DE)**

(56) References cited:
**WO-A1-2009/021518     WO-A2-03/000927
US-A- 5 834 193**

• **AGNEL J. SFEIR ET AL: "Telomere-End Processing", MOLECULAR CELL., vol. 18, no. 1, 1 April 2005 (2005-04-01), pages 131-138, XP055236132, US ISSN: 1097-2765, DOI: 10.1016/j.molcel.2005.02.035**
• **NADUPARAMBIL K. JACOB ET AL: "G-overhangs dynamics at Tetrahymena telomeres", THE EMBO JOURNAL, vol. 20, no. 15, 1 January 2001 (2001-01-01), pages 4299-4308, XP055236118,**
• **Y. ZHAO ET AL: "Quantitative telomeric overhang determination using a double-strand specific nuclease", NUCLEIC ACIDS RESEARCH, vol. 36, no. 3, 1 February 2008 (2008-02-01), pages e14-e14, XP055236143, GB ISSN: 0305-1048, DOI: 10.1093/nar/gkm1063**
• **NEAL S. YOUNG.: 'Telomere Biology and Telomere Diseases: Implications for Practice and Research' HEMATOLOGY 2010, pages 30 - 35, XP055199427**
• **SANDRA ZIELKE ET AL.: 'Telomeres and Telomerase Activity in Scleractinian Corals and Symbiodinium spp.' BIOL. BULL. vol. 218, no. 2, 2010, pages 113 - 121, XP055199432 Retrieved from the Internet: <URL:http://www. biolbull.Org/content/218/2/113.full>**
• **BAOMIN LI ET AL.: 'Sequence-specific processing of telomeric 3' overhangs by the Werner syndrome protein exonuclease activity.' AGING vol. 1, no. 3, 2009, pages 289 - 302, XP055199434**

## SEQUENCE LISTING

<110> CHIRYASOVA, Elena Andreyevna

<120> Method for the quantitative analysis of the G-strand terminal nucleotides of human telomeric DNA

<130> CHIRTNP-1

<150> RU 2012143263/10

<151> 2012-10-10

<160> 11

<210> 1

<211> 43

<212> DNA

<213> Artificial Sequence

<220>

<223> primer for synthesis of the polydeoxyadenylic minus copies of telomeric G-overhangs and for the adapter sequence inclusion

<400> 1

ctcactgaca cactatggtt tttttttttt tttttttttt ttt          43

<210> 2

<211> 43

<212> DNA

<213> Artificial Sequence

<220>

<223> primer for synthesis of the polythymidylic minus copies of telomeric G-overhangs and for the adapter sequence inclusion

<400> 2

ctcactgaca cactatggaa aaaaaaaaaa aaaaaaaaaa aaa          43

<210> 3

<211> 18

<212> DNA

<213> Artificial Sequence

<220>

<223> adapter primer for final amplification of the minus strands of telomeric G-overhangs

<400> 3

ctcactgaca cactatgg          18

<210> 4

<211> 30

<212> DNA

<213> Artificial Sequence

<220>

<223> primer for final amplification of the polydeoxyadenylic minus strands of telomeric G-overhangs

<400> 4

tttttttttt tttttttttt tttttttttt          30

<210> 5

<211> 30

<212> DNA

<213> Artificial Sequence

<220>

<223> primer for final amplification of the polythymidylic minus strands of telomeric G-overhangs

<400> 5

aaaaaaaaaa aaaaaaaaaa aaaaaaaaaa          30

<210> 6

<211> 10

<212> DNA

<213> Artificial Sequence

<220>

<223> fluorescent-labeled probe for duplex-specific nuclease analysis of the amplificated minus strands of telomeric G-overhangs for identification of TAG terminal triplet

<400> 6

ggttagcccc        10

<210> 7

<211> 10

<212> DNA

<213> Artificial Sequence

<220>

<223> fluorescent-labeled probe for duplex-specific nuclease analysis of the amplificated minus strands of telomeric G-overhangs for identification of AGG terminal triplet

<400> 7

gttaggcccc        10

<210> 8

<211> 10

<212> DNA

<213> Artificial Sequence

<220>

<223> fluorescent-labeled probe for duplex-specific nuclease analysis of the amplificated minus strands of telomeric G-overhangs for identification of GGG terminal triplet

<400> 8

ttagggcccc        10

<210> 9

<211> 10

<212> DNA

<213> Artificial Sequence

<220>

<223> fluorescent-labeled probe for duplex-specific nuclease analysis of the amplificated minus strands of telomeric G-overhangs for identification of GGT terminal triplet

<400> 9

tagggtcccc        10

<210> 10

<211> 10

<212> DNA

<213> Artificial Sequence

<220>

<223> fluorescent-labeled probe for duplex-specific nuclease analysis of the amplificated minus strands of telomeric G-overhangs for identification of GTT terminal triplet

<400> 10

agggttcccc        10

<210> 11

<211> 10

<212> DNA

<213> Artificial Sequence

<220>

<223> fluorescent-labeled probe for duplex-specific nuclease analysis of the amplificated minus strands of telomeric G-overhangs for identification of TTA terminal triplet

<400> 11

gggttacccc        10

**Description**

**The abstract**

The invention is intended for the revealing of changes in the nucleotide endings of telomeric DNA regions of the chromosomes during a cell cycle. The method is based on the new approach without use of ligation procedure. The method includes stages of the preliminary extraction of genomic DNA, the precipitation of the single-stranded fraction of telomeric G-overhangs and the following amplification of their negative strands with the duplex-specific nuclease analysis. The amplification stages include the modification of 3'-end of telomeric G-overhangs by means of a terminal deoxynucleotidyl transferase enzyme and are carried out using a set of primers presented in SEQ ID NO: 1-5. The duplex-specific analysis is based on hybridization of the negative strands of overhangs with a set of specific fluorescent-labeled signal probes (SEQ ID NO: 6-11) which corresponding to six possible variants of the G-strand nucleotide endings of human telomeric DNA. The degree of increasing levels of florescence under the condition of signal probe cleavage in the presence of duplex-specific nuclease enzyme is used as the criterion of the presence of corresponding variant of telomeric DNA termini and as a measure of its quantitative content. The Terminal Nucleotide Profiles obtained for the percentage content of the G-strand terminal triplets of telomeric DNA are used as the proliferative markers specifying for degree of the cell division activation and determining the duration of cell cycle. The invention provides the increase of sensitivity and accuracy of quantitative analysis of the terminal nucleotides in comparison with methods based on the ligation procedure. The method allows receiving the diagnostic results in immunology, oncology, transplantology, cosmetology, dermatology, gerontology, cellular biotechnology and other areas interested in an assessment of the proliferative status of cells and methods of its directed control.

## FIELD OF THE INVENTION

The invention belongs to the field of genetics and cell molecular biology, in particular, to molecular diagnostics of the processes occurring in the terminal regions of chromosomal telomeres during a cell cycle. The declared method is used for the construction of Terminal Nucleotide Profiles (TNP) of the G-strand of human telomeric DNA displaying their quantitative percentage ratio. These profiles are used as proliferative markers indicating the degree of activation or inhibition of the cell division processes and determine the duration of cell cycle.

The invention is the type of DNA-diagnostics of the proliferative status of cells, tissues and human organism as a whole. The method can find the application in diagnostics and treatment of the oncologic diseases, in the definition and monitoring of the immune status, in gerontology, cosmetology, dermatology, transplantology, cellular biotechnology and other fields which requires of controlling and target influence on a cell division.

## BACKGROUND OF THE INVENTION

The object of the invention researches are the single-stranded areas of the G-strand of human telomeric DNA, called by "G-overhangs". Till present time two methods of identification of the terminal nucleotides of G-overhangs based on a principle of ligation are known. These methods have the essential methodological restrictions which limited by impossibility of a direct ligation to the free 3'-end of single-stranded telomeric overhang.

In 2001 the method Ligation-mediated primer extension protocol was offered for the studies of elementary organism Tetrahymena thermophila. This method can allow to measure of the length and determinate the nucleotide endings of G-overhangs, containing telomeric repeats TTGGGG. The main information is present in article: Naduparambil K. Jacob, Rose Skopp and Carolyn M. Price. G-overhang dynamics at Tetrahymena telomeres. The EMBO Journal, Vol. 20, No.15, 2001, pp. 4299-4308. The impossibility of a direct ligation of the single-stranded 3'-overhang leads this method to using the ligation of universal sequence Unique oligo which preliminarily hybridizes with complementary region of Guide oligo sequences. The authors had been developed the six variants of the $^{32}$P-labeled Guide oligos which containing the sequence complementary to Unique oligo and the five-nucleotide sequence corresponding to specific terminus of G-overhang.

According to reporting method at first the Unique oligo and the Guide oligo sequences were mixed in equimolar concentration. Then the received duplexes carrying a single-stranded region from 5 nucleotides of one of six variants of telomeric repeat were added to the analyzed sample and carried out a ligation by means of T4 DNA ligase. A ligation reaction was made at 16° overnight. After removing of not ligated duplexes was carried out the completion of 3'-end of the Guide oligo sequences on all length of telomeric overhang templates using T4 DNA polymerase. The criterion of the presence of specific variant of overhang's termini was presence of the conforming fragments of labeled DNA which could be synthesized only under the condition of a ligation and full conformity between 5'-end of the Unique oligo sequences and 3'-end of the telomeric overhang. The presence of these fragments was quantitative valued by the label presence in polyacrylamide gel.

The critical moment for the achievement of ligation efficiency and for success of the analysis is the formation of nick-like structure between 5'-end of the Unique oligo sequences and 3'-end of the overhang which is linked with using of ligase. For formation a nick-like structure it is necessary that five-nucleotide region of the Guide oligo sequence which complementary to overhang telomeric repeat was annealed on its termini. In spite of the short dimensions of Tetrahymena's G-overhangs from 14 to 27 nt the probability of annealing more to the left of a terminal priming site is not excluded. Last factor, considering a dimensional double-stranded configuration of duplex which formed by the Unique oligo and the Guide oligo sequences, can disturb to further annealing of telomeric five-nucleotide region on the terminal priming site of overhang template located more to the right. This dependence of efficiency from the features of annealing and ligation is the main limiting factor of this method.

The unique method used for identification of the terminal nucleotides of human chromosomes, containing telomeric repeats TTAGGG, was method the Single Telomere length analysis (STELA), offered Agnel J. Sfeir et al. (2005, 2006). The main information is resumed in article: Agnel J. Sfeir, Weihang Chai, Jerry W. Shay and Woodring E. Wright. Telomere-End Processing: the Terminal Nucleotides of Human Chromosomes. Molecular Cell, Vol.18, 2005, pp. 131-138.

The methods allowing to investigate the terminal nucleotides of chromosome ends for both DNA strands were offered in this article. For studying of the terminal nucleotides of C-rich strand was used the method Primer-Ligation Assay and also the modification of method Single Telomere length analysis (C strand STELA) which are based on a ligation of the free 5'-end of C-strand. The great difficulty is connected with studies of the telomeric G-strand as the direct ligation of single-stranded 3'-end is impossible. In this connection the conforming modification of STELA method designed for research of the telomeric G-strand (G strand STELA) was offered in this article. This method is the closest analogue of the declared invention.

Due to impossibility of ligation of the single-stranded 3-'end of telomeric overhang, the procedure of annealing a Guide platform artificial sequence [3'-AATCCC-5']$_{10}$ on the G-strand overhang template was offered like at the previous method. But unlike a method offered N.K. Jacob et al., the annealing of a platform in length of 60 nucleotides to the 3'-overhang template was passed at the first stage before hybridization with other additional sequences. According to authors it provided the formation of artificial 5'-overhangs which allowed to create the further ligation between synthetic 5'-phosphorylated cartridges (G telorettes) and 3'-end of the telomeric overhang. Each cartridge included a region in length 7 nt corresponding to one of six variants of the G-overhang nucleotide endings and sequence complementary to universal reverse G teltail primer. The ligation of each of six variants of the cartridges was carried out only at its full conformity between 5'-end and 3'-end of the investigated G-rich strands. For the following amplification the reverse G teltail primer and the forward universal subtelomeric primer which specific to a region of subtelomeric chromosomal area were used.

The amplification of the ligated samples led to the formation of various length's products. They were analyzed in gel by means of the Southern blotting and served as a measure of the presence and quantitative measurement of specific variants of the G-overhang nucleotide endings. For this purpose the fragments of DNA was parted in the denaturing agarose gel, transferred on the nylon filter and hybridized with $^{32}$P-labeled subtelomeric probe which formed in a PCR by means of the forward and the reverse subtelomeric primers.

The essential restriction of this method and of the method offered for the research of Tetrahymena is the dependence of its efficiency from the annealing's conditions of the sequence complementary to G-overhang. Thus an indispensable condition is the terminal annealing of Guide platform sequence on the overhang's termini with formation of artificial 5'-overhangs which producing depends from the hybridization conditions, the length of platform and overhang's matrix.

At the equal cases of the hybridization between 3'-overhangs and platform the success of the adequate analysis of different lengthy populations of overhangs is represented difficult. The efficiency of the analysis is defined by a condition at which at least one unit of a platform should form an artificial 5'-overhang of optimum length not less than 6-7 nt. That is defined by the length of the telomeric sequence in each cartridge. At the reduction of 5'-overhang's length to 1-3 nt, the efficiency of following ligation at the declared temperature in 35°C reduce to absence. In this case the advantage at the ligation will be received by those cartridges which are annealed with the formation of 5'-overhangs in the length of 5 nt and more. Thus in the most unfavorable conditions for the following ligation there are the overhangs terminating on triplets GGG, AGG and TAG.

Thus it is necessary to consider that for hybridization of the platform with the telomeric overhang at the declared temperature in 70°C the forming 5'-overhang should be in the length no more than 30 nt. The larger length of 5'-overhang will create the conditions for unstable annealing of platform. In this case the bigger advantage at the ligation will be received by shorter overhangs in length 40-50 nucleotides than longer. Only this length of 3'-overhang provides the unequivocal formation of 5'-overhang, which sufficient for the further successful analysis.

The difficult moment in this procedure is the analysis of shortest overhangs less than 30 nucleotides because of the formation of an unstable duplex at the hybridization with high temperature in 70°C is represented. This restriction is particulate remove at the reduction of hybridization temperature to room temperature that was carried out by authors much later in 2009. However in this study the method G strand STELA was used only for the definition of telomere length without identification of the terminal nucleotides [Yong Zhao, Agnel J. Sfeir et al. Telomere extension occurs at most chromosome ends and is uncoupled from fill-in in human cancer cells. Cell. Vol. 138, No.3, 2009, pp. 463-475].

The annealing of the platform which can lead to formation of residual 3'-overhang is possible. The possibility of its formation rises due to primary annealing of full-length platform sequences at the declared high temperature of hybridization in 70°C. Thus always will be present such variants of hybridization in which the site of priming of the terminal telomeric repeat AATCCC-5' of platform is corresponded to the complementary full-length last repeat TTAGGG of telomeric overhang. The G-overhangs terminating on triplet GGG due to formation of blunt 3'-end are in risk to remain unligated and completely to fall out of the analysis. Possible this reason is connect to the diminished frequency of occurrence of this variant of nucleotide ending which was got by authors as a result of the ligation artefacts. In similar hard conditions there are the overhangs terminating on GGT, GTT and TTA triplets as possible to form too small residual 3'-region, insufficient for the annealing of one more platform unit and for the following ligation.

The significant part of the telomeric G-overhangs is presented by the single-stranded fragments in length more than hundred and even some hundreds nucleotides. At length over 60 nt the annealing of several platform units on all extent of the telomeric overhang is probable. Thus the next hybridization possibilities are not excluded: a) the blunt end formation especially if the investigated overhang terminates on triplet GGG-3' that is defined by termination of the platform on triplet 5'-CCC; b) the formation of residual 3'-overhang with the minimum length and with the melting point much less than chosen by authors for hybridization and ligation; c) the formation of critically small 5'-overhangs with length of 1-3 nt insufficient for the following annealing and ligation of G-telorettes.

The listed factors can lead to formation of short 5'-overhangs, insufficient for the following analysis or to its total absence. Thus the further study for a part of telomeric overhangs becomes impossible and their ligation signal appears essentially diminished. Thereby the main disadvantage of the considered method consists that various lengthy templates of overhangs ligate with different efficiency. At the equal cases the hybridization of the one templates get advantage and the ligation of others templates can be complicated.

The following feature of G strand STELA method which can lead to the essential distortion of result of the analysis, is the efficiency dependence of final PCR from a priming site of forward subtelomeric primer XpYpE2. The sequence 5'-TTGTCTCAGGGTCCTAGTG-3' of this primer according to program of the analysis of nucleotide sequences BLAST Nucleotide NCBI is present at subtelomeric area of chromosomes X and Y. Other human chromosomes can have the priming sites in the subtelomeric area which differ on the degree of homology with primer sequence, and, hence, on the competitive abilities in PCR. In this case the advantage at the amplification will be received the telomeric DNA of those chromosomes which have the greatest amount of the most homologous sites. In the most favorable conditions of amplification there is DNA of chromosomes X and Y (100% of a homology in the subtelomeric area). The telomeric DNA of other chromosomes having lower homology with XpYpE2 primer has a risk worse to amplificate at high temperature of annealing in 58°C. It follows that G-overhangs belonging to different chromosomes are amplified with different efficiency that should be reflected on the analysis result.

Thus the success of application of the considered method depends on the degree of degradation of telomeric DNA. The final amplification can be realizable only provided that the region between the forward subtelomeric primer and the reverse G teltail primer does not contain the double-stranded breaks. The telomeric areas reaching lengths of the several thousand of nucleotides, can be exposed to the destruction in the process of DNA extraction. The possibility of the integrity destruction of subtelomeric chromosomal area is not excluded also during the treating of genomic DNA by restrictase *Eco*RI at the initial stage of analysis.

The illustration that ligation and amplification stages during the G strand STELA method each time occurred variously is the receiving of electrophoresis results. The authors got the various panels of DNA bands at the repeats of analysis from 2 to 8 times for each variant of the G-overhang nucleotide endings. The disadvantages of the considered method which bound to the presence of artefacts during the ligation and the amplification procedure can be essentially reflected in the analysis result. They can lead to incorrect definition of the frequencies of occurrence of possible variants of the G-overhang nucleotide endings. It can lead to the construction of incorrect Terminal Nucleotide Profile of the G-strand of telomeric DNA playing a key role in the definition of proliferative status of cells.

## DISCLOSING OF THE INVENTION

The main technical task of the invention is the working out of a new effective method of study of the terminal nucleotides of G-rich strand of human telomeric DNA for the elimination of disadvantages of existing methods. The technical result of the invention is the rising of specificity and test-sensitivity of analysis concluding in the possibility of successful research of the total fraction of G-overhangs having different length and the obtaining of correct Terminal Nucleotide Profiles of human telomeric DNA. The main task consists in the using of application to studying of the terminal nucleotides of DNA comprising the combination of telomeric DNA amplification with the following duplex-specific analysis.

The essence of the invention consists in the creation of synthetic fluorescent-labeled signal probes which corresponding to six possible variants of the telomeric DNA termini. The presence or the absence of these variants of terminal nucleotides reveals during the hybridization of signal probes with studied telomeric DNA and their cleavage by means of the duplex-specific nuclease enzyme. The probe cleavages occur only under the condition of its full complementary conformity to the investigated sequence. It provides high specificity of analysis and leads to increasing of the specific fluorescent signal in tubes which is registered by fluorimeter. The degree of increasing fluorescent signal of probes conforming to six possible variants of the telomeric DNA termini is presented like not only qualitative criterion of their presence, but also like a measure of their quantitative ratio.

The declared method includes the possible means of registration and interpreting of the analysis results. It is offered to represent the result of fluorescent signals valuation in the form of bar diagram showing the percentage of the specific variants of terminal nucleotide triplets of telomeric G-overhangs and called by "Terminal Nucleotide Profile of the G-strand of telomeric DNA».

The basic characteristic of the declared method is defined by specificity of realization of the duplex-specific analysis in researches of human telomeric DNA. The Duplex-specific analysis or the DSNP-analysis (Duplex-specific nuclease preference analysis) is based on the use of duplex-specific nuclease (DSN) enzyme from a hepatopancreas of Kamchatka crab having the specific feature to cleavage the double-stranded DNA and to remain the single-stranded DNA. The DSNP-method was developed by the Russian scientists for the analysis of single nucleotide polymorphism of genes and successfully used for studying of point gene mutations [Shagin D.A. et al. A Novel Method for SNP Detection Using a New Duplex-Specific Nuclease from Crab Hepatopancreas. Genome Research, 2002, Vol.12, pp.1935-1942].

It is shown that the efficiency of DSN-hydrolysis of perfect DNA-duplexes considerably higher than imperfect containing at least one not coupled nucleotide base. This feature of the duplex-specific nuclease has laid down in a basis of the developed method for detection of terminal nucleotide polymorphism of the G-strand of human telomeric DNA using DSNP-analysis.

The general principle of the DSNP-analysis is concluded to use the synthetic signal probes in short length of 10-12 nucleotides carrying the donor of fluorescence on 5'-end and a fluorescence quencher on the opposite 3'-end. At the complementary biding of a signal probe and investigated DNA-target the nuclease activity of DSN leads to burning a detected fluorescent signal. The high test-sensitivity in the connection with the ability of duplex-specific nuclease to distinguish a variation in one nucleotide is shown. For detection of the one-nucleotide polymorphisms at the point mutations of genes the investigated region of DNA amplificate using polymerase strand reaction, and then a PCR product admix with probes and incubate in the presence of DSN enzyme.

The essential difference of the declared method from the classical duplex-specific analysis of nucleotide polymorphism is that it is applied to research of the terminal regions of tandemno-repeating telomeric DNA, but not for the heteronucleotide DNA sequence of genes. This property of the research object has defined the principal modification of amplification stages of the investigated telomeric DNA by a method of polymerase strand reaction using a special set of primers presented in sequences SEQ ID NO: 1-5.

The terminal location of the 3'-end of G-overhang does not allow to use a usual reverse primer for its amplification. This fact has caused of the use of artificial tailing of 3'-end using terminal deoxynucleotidyl transferase and mononucleotides that was reflected in the structure of signal probes. The set from six probes presented in SEQ ID NO: 6-11 in which the position of mismatch discrimination located on the distance of 4-5 nucleotides from 3'-end at the region of natural termination of G-overhang and the beginnings of poly (dC) tail created by a transferase.

The dCTP was chose as a building stuff because it is only one nucleotide which absent in the human telomeric repeat TTAGGG, and providing correct discrimination of all six variants of the nucleotide endings of telomeric G-strand. The combination of 5-carboxyfluorescein dye (FAM) on 5'-end and its effective quencher RTQ1 on 3'-end is offered in examples of invention realization. It has allowed to realize the quantitative analysis for all possible variants of G-overhang nucleotide endings due to intensity of the growth of green fluorescence in time. Any dyes and quenchers in the areas of corresponding fluorescence, including multichannel can be used at the designing of probes.

The efficiency of using labeling variants will be defined by spectral characteristics of the fluorophore-bound fluorescence of DNA by the current moment of the researches. Our studies in declared method have found that the nucleotide sequences of DNA have specific own fluorescence in a visible spectrum of light waves, connected with formation of general quantum-bound spectrum with a molecule of fluorescent dye. According to received results the DNA have not only the known fluorescence in UV spectrum, but also has the own multicolour integrated spectrum of the fluorophore-bound fluorescence in a visible spectrum of light waves. The specificity of this fluorescence in each colours of a visible spectrum depends on the nucleotide sequence of DNA. In this connection the nucleotide sequence of probes labeled with fluorescent dye defines the specificity of its fluorescence in the conforming spectrum of a visible light and also its fluorescent signal at the DSNP-analysis.

The basic characteristic of the fluorophore-bound fluorescent properties of DNA is a spectral code which represents the interrelation of fluorescence levels of basic colour channels in a visible spectrum of light. At the moment of studies in 2010-2011 the specificity of background fluorescence and of cleavage of used FAM-RTQ1 probes corresponded to the current spectral code of DNA fluorescence. It has the characteristic strongly expressed emission of the fluorescence of telomeric DNA in the green and red spectrums of visible light. The gradation of augmentation of the energy potential of telomeric repeats in a green spectrum in the probes terminating on a various amount of guanine was accurately displayed. It has led to necessity of using of the various concentrations of FAM-labeled probes for alignment of its background fluorescence at the examples of invention realization. Besides at the processing of results of the DSNP-analysis were used a probe-specific coefficients. They reflected the specificity of increase of the probe fluorescence at their cleavage during the DSNP-analysis. It was defined by features of the fluorophore-bound fluorescence of the probe sequences in used spectrum of light and directly depended on their nucleotide sequence (see the results of DSNP-analysis at Example 1).

The researches have shown that the spectral code of the DNA fluorescence is dynamical in time and the interrelation of the fluorescence levels of basic colour channels is the subject to fluctuations. These changes should be supervised in due time as directly reflected on the levels of the background fluorescence of probes and their specificity of its increasing fluorescence at the DSNP-analysis. The adequacy of using probes concentration and of the probe-specific coefficients at the analysis should be checked by the control research of an equimolar mixture of the synthetic analogues of overhang nucleotide endings, as described in Example 5.

**Principle scheme for quantitative analysis of the G-strand terminal nucleotides of human telomeric DNA using PCR and DSNP-analysis**

**The first stage.** The fraction of the single-stranded overhangs of telomeric DNA separate from the sample of genomic DNA received by any methods conserving it native state. For these purposes the duplex-specific nuclease enzyme is used because its efficiency was shown in study of telomeric overhangs length [Yong Zhao et al. Quantitative telomeric overhang determination using a double-strand specific nuclease. Nucleic Acids Research, 2008, Vol.36, No. 3]. The researches have shown that the use of DSN in concentration 0,2 units on each 5 $\mu$g of genomic DNA allows to the effectively cleavage of the double-stranded DNA to fragments in length no more than 10 base pairs and to conserve the native fraction of the single-stranded overhangs.

In declared method for the concentrating and purification of overhangs from the residual genomic DNA we have offered the modification of procedure using streptavidin-covered paramagnetic beads [Woodring E. Wright et al. Normal human chromosomes have long G-rich telomeric overhangs at one end. Genes & Development. 1997, Vol.11, No. 21, pp.2801-2809]. According to authors 40 $\mu$g of genomic DNA contains order 1 fmole of the telomeric termini. According to it no more than 1 pmole of the biotinylated oligonucleotide probe (CCCTAA)$_4$ and 3 $\mu$l of the streptavidin-covered magnetic beads in concentration of 10 mg/ml are enough for precipitation even the longest overhangs.

In examples of the invention realization the use of the magnetic beads with streptavidin in concentration of 1 mg/ml ("Sileks", Russia) with the efficiency of binding on average 8 pmole of biotinylated product on 10 $\mu$l beads is offered. Because the precipitation of the fraction of different length overhangs goes more slowly than binding of the short oligonucleotides with the complexes "beads-probe" we offered to use 10 $\mu$l of beads on each 20 $\mu$g of initial genomic DNA. It is enough even the considering some loss of the magnetic beads at the washings. For the preliminary cleavage of the genomic DNA we have offered the use of duplex-specific nuclease instead of restrictase *Hin*fl. Thus the binding of the native overhangs with the complementary probes preceded its preliminary incubation with the magnetic streptavidin-covered beads. The use of several washings in low-saline buffer provides the effective removal of the probes unbounded with magnetic beads at the first stage and of the residual double-stranded genomic DNA at a following stage. Such manipulations provides the time reduction of the overhangs precipitation till 1,5 hours in comparison with the initial procedure described Wright et al., where the final stage of the DNA incubation with magnetic beads was carried out overnight. At the final elution the temperature increasing to 70-75°C allows to effectively concentrate the fraction of investigated overhangs in the volume approaching for the further stage of poly(dC)-tailing. Thus the use of a triple biotin probe CTAACCCTAACCCTAACCCTA (SEQ ID NO: 12) provides the strong binding with the streptavidin on the magnetic beads at the high-temperature elution. The any hybridization and sorption approaches for obtaining of the purified fraction of single-stranded overhangs can be used, including various membranes, columns, beads or other surfaces for the immobilization and the following putting off from their surface of investigated DNA molecules.

**The second stage.** The water eluent containing the single-stranded G-overhangs, use as substrate for the terminal deoxynucleotidyl transferase which incorporated the mononucleotides to 3'-end of DNA strand. At this stage the use of dCTP as a building stuff creates of the polydeoxycytidylic sequences. Thus the field of carrying out of DSNP-analysis is the region of contact of the natural 3'-overhang terminus and the beginnings of poly(dC) tailing. The duration of synthesis is selected empirically and carried out during time sufficient for the building of the sequence which is necessary for the maintenance of efficiency of the following sedimentation of short overhangs at their purification.

**The third stage.** After standard procedure of precipitation and purification from not incorporated dCTP the DNA sample use for the similar poly(dA)/poly(dT) tailing by means of the dATP/dTTP and terminal deoxynucleotidyl transferase. The combination of the consecutive stages of polydeoxycytidylation and polydeoxyadenylation (polydeoxythymidylation) allows to separate the investigated contact of G-overhang nucleotide ending and the poly(dC) tail from a priming site at the following synthesis of negative strands. The conservation of nativity of this border is the main condition for the obtaining of adequate results of the analysis.

**The fourth stage.** At this stage make synthesis of the negative strands with use of thermostable Taq DNA polymerase and oligo $dT_{25}$ Adapter primer (SEQ ID NO: 1) in case of carrying out of the 3'-polydeoxyadenylation at the previous stage. In other case make synthesis of the negative strands with the oligo $dA_{25}$ Adapter primer (SEQ ID NO: 2) if used the polydeoxythymidylation at the third stage. The both variants of primers contain the adapter sequence (SEQ ID NO: 3) on the 5'-end. The any nontelomeric DNA sequence can be used as the adapter corresponding to the optimum temperature of annealing at the synthesis of negative strands. The amount of cycles of the negative-strand synthesis increase to 100-200 cycles that is important for the obtaining of the sufficient level of a fluorescent signal at the final DSNP-analysis. This condition is especially important at the case of a small initial quantity of analyzed DNA.

**The fifth stage.** After purification and sedimentation of the negative-strand fraction make its 3'-tailing using the terminal transferase and dATP if was used the 3'-polydeoxyadenylation of the overhang strands at the third stage. In other case the dTTP is used if was applied polydeoxythymidylation at the third stage.

**The sixth stage.** The purified fraction of the negative strands with poly(dA) tail use for the final asymmetrical PCR amplification using the adapter sequence (SEQ ID NO: 3), or similar to it, as a forward primer and oligo $dT_{30}$ primer (SEQ ID NO: 4) as the reverse primer. In the second variant the negative strands with poly(dT) tail amplificate with use of the same adapter sequence as a forward primer, but with oligo $dA_{30}$ primer (SEQ ID NO: 5) as the reverse primer. The amount of PCR cycles is selected experimentally, but no more than 18-20 so to provide the conservation of an initial interrelation of G-overhang nucleotide endings.

The use of asymmetrical PCR with an interrelation of forward primer to reverse primer 10:1 provides the predominantly synthesis of the negative strands and the conditions of its following hybridization with the fluorescent probes. Thus the domination of adapter primer leads to displacement of oligo $dT_{30}$ (oligo $dA_{30}$) primer which can particulate annealed as a forward on the poly(dA/dT) sequence located immediately for the adapter.

**The seventh stage.** The realization of DSNP-analysis. The purified PCR product separate on six equal parts and admix with the fluorescent probes in the presence of duplex-specific nuclease. The design of signal probes is selected according to specificity of investigated border between the 3'-overhang terminus and the beginning of poly(dC) sequence. The signal probes represents a synthetic oligonucleotides in length of 10-12 nucleotides, presented of the plus strand of telomeric G-overhang and corresponding to the one of six variants of its nucleotide endings (SEQ ID NO: 6-11). The structure of signal probes designed for the duplex-specific analysis, assumes the presence of a molecule of fluorescence donor on 5'/3'-end and corresponding fluorescence quencher on the inverse 3'/5'-end. In examples of the invention realization the combination of dye FAM and quencher RTQ1 was used. The use of other dyes in the combination with quenchers at the corresponding fluorescence channels, including multichannel registration is possible.

After inserting of probes into the corresponding tube containing the investigated sample, before addition of enzyme DSN, make measurement of the background fluorescent signal which then is subtracted at the normalization of fluorescence signal during the probe cleavage. The hydrolysis of signal probes is carried out in the presence of duplex-specific nuclease only at its full complementary conformity to a region of amplificated negative strands of G-overhangs. A probe cleavage occurs mainly between the fourth and fifth or fifth and sixth nucleotides (Anisimova V.E. et al. Isolation, characterisation and molecular cloning of Duplex-Specific Nuclease from the hepatopancreas of Kamchatka crab. BMC Biochemistry, 2008, Vol. 9, №14). The dimensional dissociation of dye and quencher leads to increasing of fluorescence in the corresponding channel of detection. For dye FAM it is correspond to 520 nanometers of wave length in a green spectrum of visible light.

The increase of fluorescence in the tube with known probe testifies to presence of the corresponding G-overhang nucleotide ending. Thus not only the qualitative test of the presence of specific nucleotide endings is possible, but also the semiquantitative measurement of their content. It is very important for the monitoring of interrelation changes of the various variants of G-overhang nucleotide endings during a cell cycle. The measurement of a fluorescent signal carry out in the tubes by any known kinds of devices designed for this purpose (spectrofluorimeters, photometers-fluorimeters, fluorescent scanners and detectors, fluorescent stereomicroscopes, etc.).

The interpreting of the results of probe cleavage includes a stage of fluorescent signal normalization. It consists in the subtraction of initial background before carrying out of the analysis, and the multiplication of received signal value with a probe-specific coefficient empirically calculated for the each fluorescent probe. The coefficients calculate by plotting of the fluorescence growth curve in time for each probe during the control research of an equimolar mixture of telomeric overhangs. For this purpose use of the synthetic analogues of G-overhangs which has passed the all analysis stages, since the synthesis of poly(dC) tail and concluding the final amplification of its negative strands, similarly to Example 5.

The coefficients are specific to each probe separately and each combination of "dye-quencher". They display the rate of probe cleavage which is constant during the analysis period and correspond to current spectral code of the fluorophore-bound DNA fluorescence in visible light. As described earlier, the coefficients reflect the specificity of energy potential of telomeric repeats which are a part of signal probes. Basically it is defined by a spectral code of the guanine fluorescence at the moment of current research. At the examples of the invention realization the coefficients for normalization for six variants of signal probes labeled by a combination of dye FAM and quencher RTQ1 after one and two hours of the DSNP-analysis are described in Table 1.

**Table 1. Coefficients for normalization of the fluorescence signals for probes FAM-RTQ1 at the DSNP-analysis**

| Variant of the signal probe and detected terminal triplet | Final concentration in reaction | 1 hour of the analysis | 2 hours of the analysis |
|---|---|---|---|
| 1. GGTT**AG**CCCC | 1,1 μM | 1 | 1 |
| 2. GTT**AGG**CCCC | 0,8 μM | 0,765 | 0,698 |
| 3. TTA**GGG**CCCC | 0,4 μM | 0,612 | 0,528 |
| 4. TAG**GGT**CCCC | 0,8 μM | 0,221 | 0,178 |
| 5. AGG**GTT**CCCC | 1,5 μM | 3,745 | 3,247 |
| 6. GGG**TTA**CCCC | 1,5 μM | 2,967 | 2,491 |

The changes of the spectral code of DNA fluorescence, the other probe concentration like other variants of labeling demand a new calculation of the signal normalization coefficients for each probe. Therefore the periodic research of a control sample of an equimolar mixture of the synthetic analogues of overhang nucleotide endings for the purpose of verification of signal normalization coefficients is obligatory. It has the special importance in the conditions of unstable spectral properties of DNA.

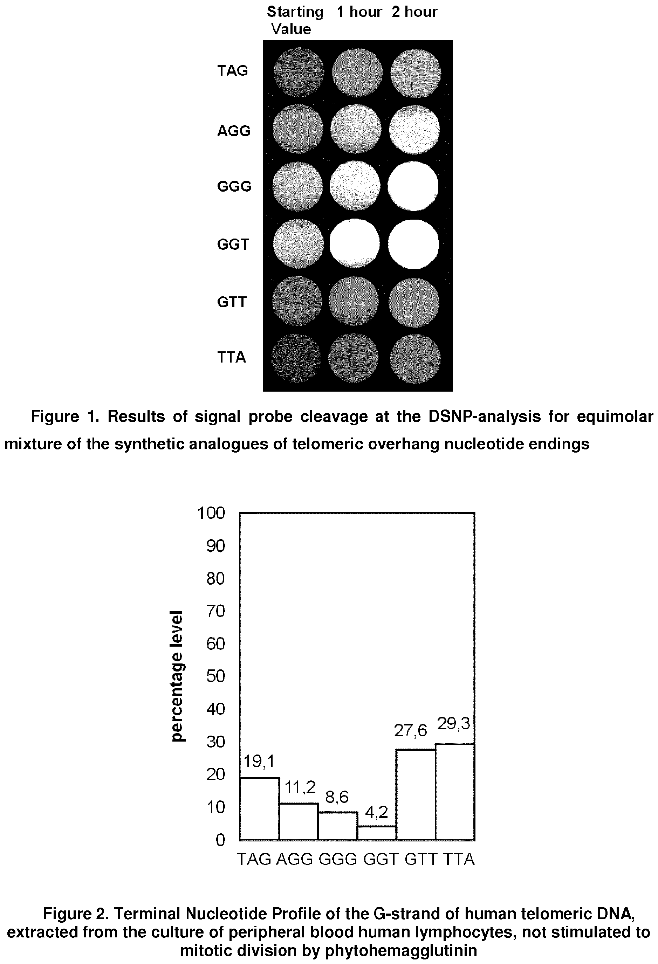

The total value of the signal for each probe is calculated with use of normalization coefficients for one and two hours and then averaged. The result is presented in the form of bar diagram displaying the average percentage of received signals of each probe from a total level of a signal received from all probes. This bar diagram of a percentage ratio of the variants of terminal nucleotide triplets of telomeric G-overhangs, called by the Terminal Nucleotide Profile (TNP) of the G-strand of telomeric DNA (Fig. 2).

The essence of the invention consists in use of a new approach for quantitative analysis of the G-strand terminal nucleotides of telomeric DNA. The declared method differs from known methods that: a) for final amplification use the polydeoxyadenylic (polythymidylic) copies of the negative strands containing the adapter sequence which used as the forward primer taken in excess to the reverse poly(dT)/poly(dA) primer; b) the results analyze by means of the hybridization with fluorescent-labeled signal probes in the presence of duplex-specific nuclease; c) the results register on a degree of the probe cleavage and of the fluorescence increase by means of any hardware-software complexes, allowing to display the fluorescence in tubes on the screen of computer and quantitatively define the signal level in each of them.

The comparative analysis with a prototype allows to make a conclusion that the declared method essentially differs from the described method G strand STELA which offered Agnel J. Sfeir et al. because is based on other approach with application of the duplex-specific nuclease enzyme without use of ligation procedure. The declared method differs from the possibility of effective analysis of all fractions of telomeric G-overhangs of any length and different nucleotide endings. This fact is the main technical result of declared invention expressed in rising of the specificity and sensitivity of the analysis in comparison with a prototype. In this connection the declared method allows to define an interrelation of the triplet variants of telomeric overhang nucleotide endings more precisely. The construction of correct profile of the G-strand terminal nucleotides of telomeric DNA is a marker of cell division processes. Thus the declared method can become the new instrument of fundamental researches in cell biology and the decision of the applied problems in medicine and biotechnology.

## SEQUENCE LISTING FREE TEXT

The declared method includes the amplification of the negative strands of telomeric G-overhangs by means of specific oligonucleotide primers presented in Table 2, and the following duplex-specific analysis with use of specific oligonucleotide probes described in Table 3. The primers SEQ ID NO: 1 and 2 provide the inclusion of adapter sequence SEQ ID NO: 3 to synthesized negative-strands of polydeoxyadenylic (polythymidylic) templates of telomeric G-overhangs (stage 4 of the analysis). The primers SEQ ID NO: 4 and 5 in a combination with the adapter primer SEQ ID NO: 3 are used for final amplification PCR of the polydeoxyadenylic (polythymidylic) negative strands of telomeric G-overhangs (stage 6 of the analysis). The oligonucleotide probes having SEQ ID NO: 12 provide the isolation of a fraction of telomeric G-overhangs with the aid of magnetic beads at the initial stage of analysis.

**Table 2. List of the deoxyribo-oligonucleotides used as the primers for polymerase chain reaction**

| SEQ ID | Name of the primer | Sequence in 5'-3' direction |
|---|---|---|
| NO: 1 | Oligo $dT_{25}$ Adapter Primer | CTCACTGACACACTATGGTTTTTTTTTTTTTTTTTTTTTTTTT |
| NO: 2 | Oligo $dA_{25}$ Adapter Primer | CTCACTGACACACTATGGAAAAAAAAAAAAAAAAAAAAAAAAA |
| NO: 3 | Adapter Primer | CTCACTGACACACTATGG |
| NO: 4 | Oligo $dT_{30}$ Primer | TTTTTTTTTTTTTTTTTTTTTTTTTTTTTT |
| NO: 5 | Oligo $dA_{30}$ Primer | AAAAAAAAAAAAAAAAAAAAAAAAAAAAAA |

**Table 3. List of the deoxyribo-oligonucleotides used as the fluorescent-labeled probes at the DSNP-analysis**

| SEQ ID | Name of the probe | Sequence in 5'-3' direction |
|---|---|---|
| NO: 6 | Probe TAG | F/Q -GGTTAGCCCC- Q/F |
| NO: 7 | Probe AGG | F/Q -GTTAGGCCCC- Q/F |
| NO: 8 | Probe GGG | F/Q -TTAGGGCCCC- Q/F |
| NO: 9 | Probe GGT | F/Q -TAGGGTCCCC- Q/F |
| NO: 10 | Probe GTT | F/Q -AGGGTTCCCC- Q/F |
| NO: 11 | Probe TTA | F/Q -GGGTTACCCC- Q/F |

The note. F - fluorescent label on 5' or 3'-end, Q - quencher of fluorescence on 3' or 5'-end.

The fluorescent-labeled probes SEQ ID NO:6-11 are used for carrying out of duplex-specific analysis of the amplificated templates of G-overhangs negative strands for the purpose of identification of the terminal triplets TAG, AGG, GGG, GGT, GTT and TTA (stage 7 of the analysis).

## BRIEF DESCRIPTION OF THE DRAWINGS

**Figure 1.** Results of signal probe cleavage at the DSNP-analysis for equimolar mixture of the synthetic analogues of telomeric overhang nucleotide endings (Example 5). It presents the rows of consecutive pictures of tubes in the green spectrum, received by fluorescent detector FDG-001. The representation of tubes in the view of round regions corresponds to measurements of the optical density in program LabWorks (UVP, England) are located on the single lines for each variant of a probe. The first line corresponds to TAG variant of the G-overhang nucleotide endings of telomeric DNA, and last - to TTA variant. In each line the first region corresponds to starting value of the background, the second and third regions - to levels of a fluorescent signal after one and two hours of the DSNP-analysis accordingly.

**Figure 2.** Terminal Nucleotide Profile of the G-strand of human telomeric DNA, extracted from the culture of peripheral blood human lymphocytes, not stimulated to mitotic division by phytohemagglutinin (Example 1). It shows the final data processing of DSNP-analysis which represent a percentage ratio of six possible variants of the terminal nucleotide triplets of investigated population of telomeric G-overhangs.

**Figure 3.** Terminal Nucleotide Profile of the G-strand of human telomeric DNA, extracted from the culture of peripheral blood human lymphocytes, stimulated to mitotic division by phytohemagglutinin during 2 hours (Example 2).

**Figure 4.** Terminal Nucleotide Profile of the G-strand of human telomeric DNA, extracted from the culture of peripheral blood human lymphocytes, stimulated to mitotic division by phytohemagglutinin during 4 hours (Example 3).

**Figure 5.** Terminal Nucleotide Profile of the G-strand of human telomeric DNA, extracted from the culture of peripheral blood human lymphocytes, stimulated to mitotic division by phytohemagglutinin during 11 hours (Example 4).

**Figure 6.** Terminal Nucleotide Profile of the G-strand of telomeric DNA for equimolar mixture of the synthetic analogues of telomeric overhang nucleotide endings (Example 5).

**Figure 7.** Combined curves for the dynamics of changes of Terminal Nucleotide Profile on a time scale of PHA influence on the culture of human lymphocytes during first eleven hours.

**Figure 8.** Terminal Nucleotide Profile of the G-strand of human telomeric DNA for the leukemic culture Jurkat synchronized in the mitotic phase of cell cycle.

**Figure 9.** Terminal Nucleotide Profile of the G-strand of human telomeric DNA for the leukemic culture K-562 synchronized in the mitotic phase of cell cycle.

## EXAMPLES

### EXAMPLE 1. Analysis of the G-strand terminal nucleotides of human telomeric DNA extracted from the culture of peripheral blood lymphocytes, not stimulated to mitotic division by phytohemagglutinin (PHA).

The analysis of gene expression by a method RT-PCR has shown that the used lymphocytes culture was characterized by the low natural level of cyclin D2 and D3 expression. It testified to the weak progression of G1 phase of cell cycle conforming to early preparation for mitotic division at insignificant part of the cells. The expression of A, B and E-types of cyclin genes has not been detected that spoke about the absence of mitosis and synthetic phase of cell cycle. Thereby this culture of lymphocytes satisfied to conditions of the nondividing state and the weak activation of preparation for mitotic division at insignificant part of the cells. This information is important for the definition of Terminal Nucleotide Profile of the G-strand of telomeric DNA during of the cell cycle progression.

For precipitation of the single-stranded 3'-overhangs of telomeric G-strand used the sample of genomic DNA extracted by means of set Diatom™ DNA Prep100 ("Biokom", Russia). The DNA extraction was made according to producer references in some modification. At the first stage the incubation of sample in lysis buffer and a final stage of DNA elution made at temperature 55°C, instead of 65°C, in order to avoid DNA denaturation. Further 20 $\mu$g of genomic DNA put into a reaction mixture consisting of following components:

> 4 $\mu$l of 10X buffer for duplex-specific nuclease ("Evrogen", Russia);
> 0,8 units of duplex-specific nuclease ("Evrogen", Russia).

Reaction mixture volume leads up to 40 $\mu$l and gently mixed by vortex.

The reaction of cleavage of double-stranded DNA was made at 37°C for 2,5 hours. Then enzyme DSN inactivated to adding of DSN stop solution or using the 3-fold treating by solution of sorbent BlueSorb ("Klonogen", St.-Petersburg). For this purpose added 6 $\mu$l of sorbent to the sample, carefully mixed by vortex and precipitated a sorbent using short-term centrifuging with the following separating of the fluid phase keeping DNA.

After removal of nuclease the sample incubated with the magnetic beads carrying on the surface the oligonucleotide probes, complementary to repeats of the G-strand of telomeric DNA. For these purposes used the superparamagnetic beads covered streptavidin in concentration 1 mg/ml ("Sileks", Russia) and synthetic oligonucleotide probes consisting of SEQ ID NO: 12 and three molecules of biotin on the 5'-end ("Synthol", Russia). The experiments have shown that 10 $\mu$l of the magnetic beads bound nearby 8 pmole of a probe. This quantity is enough for a precipitation of the single-stranded overhangs. The redundancy of used quantity of beads and probes in relation to quantity of telomeric overhangs is necessary for the maintenance of binding efficiency of the magnetic beads with extended telomeric sequences in order to avoid their loss at the washings.

All manipulations with the magnetic beads were made according to producer references, using the original washing solutions and a magnetic support stand. The resuspended magnetic beads in amount of 10 $\mu$l washed using 200 $\mu$l of 0,5x washing solution three times, suspended in 100 $\mu$l volume of the same solution and incubated with 10 pmole of 3xbiotinylated probe. A conjugation of streptavidin with biotin was made at room temperature for 30 minutes. Thus the sample was gently suspended by vortex or pipet-batcher by each 5-7 minutes. Then magnetic beads washed three times using 0,5x washing solution for removal of the unbounded probe, suspended in 50 $\mu$l of 1 x washing solution and added an investigated sample of DNA after nuclease treatment.

The binding of the probes with native fraction of the single-stranded G-overhangs was carried out at room temperature for 40 minutes, accurately mixed by vortex or pipet-batcher each 5-7 minutes. After that the magnetic beads with immobilized G-overhangs washed from the residual double-stranded genomic DNA using 200 $\mu$l volume of 0,1x washing solution four times. After careful removal of residual washing buffer the magnetic beads suspended in the 20 $\mu$l volume of distilled purified water and incubated at 70-75°C for 5 minutes. A temperature elution was made twice, each time collecting the magnetic beads by magnetic stand support and pipetted of the eluent solution to new tube. The second elution carried out in 15 $\mu$l volume of the water. The joined water eluent used for reaction of 3'-polydeoxycytidylation (poly(dC) tailing) in total volume 40 $\mu$l of the reaction mixture containing:

> water eluent of overhangs;

> 8 $\mu$l of 5x buffers for TdT ("Sileks", Russia);
> 2 $\mu$l of 5 MM dCTP solution ("Sileks", Russia);
> 2,5 units of terminal deoxynucleotidyl transferase ("Sileks", Russia).

If necessary the reaction mixture volume lead up to 40 $\mu$l, using the purified distilled water. The reaction was made at 37°C for 40 minutes, delicate mixed by vortex each 10 minutes.

Without special inactivation of TdT was carried out the precipitation of DNA sample using the addition:

1 $\mu$l of glycogen solution with concentration 20 mg/ml ("Sileks", Russia);
25 $\mu$l of 7 M acetate ammonium solution;
165 $\mu$l of refrigerated 96 % ethanol.

The mixture well mixed by vortex and put in a freezer at temperature -20°C for not less than 12 hours.

The sedimentation was carried out by centrifuging at 15 000 rpm for 60 minutes. The second centrifuging was made at the same conditions for 30 minutes after delicate washing of a sediment using 300 $\mu$l of refrigerated 70 % ethanol. The sediment was dried at the open tube and dissolved in 25 $\mu$l of purified distilled water.

Further the reaction of 3'-polydeoxyadenylation (poly(dA) tailing) was carried out in the same tube, with the difference that instead of the solution dCTP added 2 $\mu$l of 5 MM dATP solution similarly described above method. The reaction of poly(dA) tailing was made at 37°C for 30 minutes and then the DNA precipitation was carried out by the described above method. A sediment was dissolved in 25 $\mu$l of purified distilled water after the final centrifuging.

The reaction of the negative-strand synthesis was made in 40 $\mu$l volume on the programmed thermocycler "Tertsik" ("DNA-technology", Russia). The sample after mixing by vortex was put in 0,5 ml PCR tube containing following components:

4 $\mu$l of 10x buffers for Taq DNA-polymerase, containing 25 mM MgCl$_2$ ("Sileks", Russia);
3 $\mu$l of 5 MM dNTP solution ("Sileks", Russia);
4 $\mu$l of 20 $\mu$M solution of primer SEQ ID NO: 1;
2,5 units of thermostable Taq DNA-polymerase ("Sileks", Russia).

The reaction mixture volume lead up to 40 $\mu$l. The synthesis was made in 4 rounds on 50 cycles everyone with a following temperature conditions: 94°C - 40 s, 43°C - 30 s, 72°C - 40 s. The initial denaturation at the first round at 95°C lasted 30 seconds, and in the following rounds was enlarged till two minutes. Finishing strand elongation at 72°C was enlarged till 3 minutes in first three rounds and till 5 minutes in a final round. Before carrying out of each synthesis round the new portion of thermostable Taq DNA-polymerase was added to the reaction mixture in amount of 2,5 units. The total number of cycles of the negative-strand synthesis has compounded 200.

After the final round of the negative-strand synthesis the DNA sample was put to 1,5 ml microtube where added equal volume of chloroformium. After intensive shaking by vortex the centrifuging at 10 000 rpm for 3 minutes was made. To the supernatant added:

1 $\mu$l of tRNA solution with concentration 10 mg/ml ("Sileks", Russia);
25 $\mu$l of 7 M solution of ammonium acetate;
165 $\mu$l of refrigerated 96 % ethanol.

The mixture carefully mixed by vortex and put in a freezer at -20°C temperature for not less than 12 hours. The use of tRNA for DNA coprecipitation has allowed to avoid the sedimentation of primers which have not incorporated into DNA strand. The using of glycogen or linear polyacrylamide is not comprehensible to these purposes, because they are precipitate of the short oligonucleotides.

The sedimentation was carried out by centrifuging at 15 000 rpm for 60 minutes. The second centrifuging was made at the same conditions for 30 minutes after delicate washing of a sediment with 300 $\mu$l of refrigerated 70 % ethanol. The sediment was dried at the open tube and dissolved in 25 $\mu$l of the purified distilled water.

The reaction of 3'-polydeoxyadenylation (poly(dA) tailing) of the negative-strand fraction was made in the same microtube where serially added:

8 $\mu$l of 5x buffers for TdT ("Sileks", Russia);
4 $\mu$l of 5 MM dATP solution ("Sileks", Russia);
15 units of terminal deoxynucleotidyl transferase ("Sileks", Russia).

The reaction mixture volume lead up to 40 $\mu$l, using the purified distilled water. The reaction was made at 37°C with delicate mixing by vortex each 10 minutes. The poly(dA) tail synthesis carried out during 30 minutes without special temperature inactivation of enzyme at the end.

The DNA precipitation was made right after the poly(dA) tailing reaction. For this serially added:

1 µl of glycogen solution with concentration 20 mg/ml ("Sileks", Russia);
25 µl of 7 M solution of ammonium acetate;
165 µl of refrigerated 96 % ethanol.

The mixture carefully mixed by vortex and put in a freezer at temperature -20°C for not less than 12 hours.
The sedimentation was carried out by centrifuging at 15 000 rpm for 60 minutes. The second centrifuging was made at the same conditions for 30 minutes after delicate washing of a sediment with 300 µl of refrigerated 70 % ethanol. The sediment was dried at the open tube and dissolved in 50 µl of the purified distilled water.
The reaction of final asymmetrical amplification of the negative overhang strands was made in two 0,5 ml microtubes in the final reaction volume 40 µl on programmed thermocycler "Tertsik" ("DNA-technology", Russia). The DNA sample after careful mixing was separated on two parts and put into two PCR tubes, each of which contained the following components:

4 µl of 10x buffers for Taq DNA polymerase, keeping 25 mM $MgCl_2$ ("Sileks", Russia);
4 µl of 5 MM dNTP solution ("Sileks", Russia);
4 µl of the solution containing a mixture of adapter primer (SEQ ID NO: 3) and Oligo $dT_{30}$ primer (SEQ ID NO: 4) in concentration 20 µM and 2 µM accordingly;
5 units of thermostable Taq DNA-polymerase ("Sileks", Russia).

The reaction mixture volume in each tube lead up to 40 µl and delicately mixed contents. The PCR amplification carried out for 20 cycles with a following temperature conditions: 94°C - 40 s, 48°C - 30 s, 72°C - 60 s. The initial denaturation was made at 95°C for 3 minutes, the final elongation of strands at 72°C was enlarged till 5 minutes. The increase of cycle number is not recommended, as can lead to the distortion of an initial interrelation of the variants of G-overhang nucleotide endings.
After carrying out of polymerase chain reaction the samples was removed from under the oil and put into 1,5 ml tubes where added equal volume of chloroformium. After intensive shaking by vortex the centrifuging at 10 000 rpm for 3 minutes was made. To the supernatant was added:

1,5 µl of glycogen solution with concentration of 20 mg/ml ("Sileks", Russia);
50 µl of 7 M solution of ammonium acetate;
329 µl of refrigerated 96 % ethanol;

The mixture was carefully mixed by vortex and put in a freezer at temperature -20°C for 1,5 hours. The incubated for more long time can lead to the undesirable excessive sedimentation of uncorporated PCR primers.
The sedimentation was made by centrifuging at 15 000 rpm for 40 minutes. The second centrifuging at the same conditions was made for 20 minutes after washing of a sediment by 70 % alcohol in volume 400 µl. The sediment was dried at the open tube and dissolved in 90 µl of the purified distilled water.
For duplex-specific (DSNP) analysis was prepared six thin-walled 0,5 ml PCR tubes ("Axygen", USA), in each of them was added:

2 µl of 10x buffers for duplex-specific nuclease ("Evrogen", Russia);
3 µl of solution containing of one of six probes presented in sequences SEQ ID NO: 6-11.

The probe oligonucleotides were synthesized by company "Synthol" (Russia) and contained a molecule of FAM on 5'-end and a quencher of fluorescence RTQ1 on the 3'-end.
The initial concentration of solutions for each probe were selected empirically. They was corresponded to following concentration: 10 µM for SEQ ID NO: 10 and SEQ ID NO: 11; 7,5 µM for SEQ ID NO: 6; 5 µM for SEQ ID NO: 7 and SEQ ID NO: 9; 2,5 µM for SEQ ID NO: 8. It is necessary to notice that the resulted concentration of probes, and conditions of carrying out and processing of the DSNP-analysis results was satisfied to the conditions of a spectral code of fluorophore-bound DNA fluorescence which existing at the moment of researches (December, 2010-October, 2011).
The possible essential changes of a spectral DNA code demand recalculation of solutions concentrations and coefficients of fluorescent signals normalization for each probe in Table 1. The use of other dyes in distinct from green fluorescence spectrums also demands a separate definition of probes concentration and coefficients of signals normalization according to current spectral code of DNA fluorescence. These definitions are carried out during the study of the control sample representing an equimolar mixture of various nucleotide endings of synthetic telomeric overhangs (Example 5). In connection with possibilities of spectral instability of fluorescent properties of DNA the periodic research of a control sample is the extremely expedient and necessary.
For DSNP-analysis insert 15 µl of carefully mixed aliquot of the DNA sample into each tube containing the DSN-buffer and a specific probe. After mixing and short-term denaturation of the sample at 94°C were made a cooling to room temperature for probe annealing and measurement of a background signal for each tube. The measuring was made with use of the hardware-software complex consisting of fluorescent detector FDG-001 (joint device-engineering with Institute of Theoretical and Experimental Biophysics, Pushchino, Russia) and program LabWorks 4.6 (UVP Biolmaging Systems, England).

The detector FDG-001 is designed for revealing of fluorescent signals in different spectrums of a visible light. The green channel of detection was corresponded to the spectral characteristics of dye FAM and had a range of exciting radiance equal 450-490 nanometers, and registered radiance - 510-560 nanometers. For fluorescence excitation of optical label the tubes with probes was evenly shined with the light-emitting diodes of dark blue light of corresponding wave length. The blue light, passing through a green interferential light filter, got to an objective of monochrome videocamera WAT-120N (Watec Co Ltd, Japan) for obtaining of graphical images of fluorescent tubes. The video card Studio™MovieBox (Pinnacle Systems GmbH, Germany) was transformed the television signal to digital form and allowed to display the tube images on the screen of computer and save it in the graphic file view.

In each tube was added 0,125 units of duplex-specific nuclease (DSN) after saving of the images of a background signal for each probe. The cleavage of probes was carried out at 37°C for two hours, delicately mixing by vortex each 30 minutes. After one and two hours of the DSNP analysis was made the video capture of each tube in the view of fixed round area like image on Fig.1 received at example 5.

The rows of consecutive pictures of tubes in a green spectrum was analyzed in program LabWorks, allowing to make the measurements of optical density level of the corresponding area of tube image. The levels of the mean optical density was received by the program in standard units and served as an intensity measure of probe fluorescent signals in the corresponding tubes. Any fluorescence detection devices and software complexes can applicate for measurement of the levels of fluorescent signals.

The data processing was included following stages.

1st stage: the subtraction of a background signal and obtaining of the level of fluorescent signal for each probe after 1 and 2 hours of DSNP analysis;

2nd stage: the normalization of levels of fluorescent signal comprising the multiplication by the specific coefficient corresponding to each probe from Table 1 for 1 and 2 hours of DSNP analysis;

3rd stage: the definition of levels of fluorescent signals for each probe in percentage after 1 and 2 hours of DSNP analysis. Stages 1-3 of data processing are presented in Table 4.

**Table 4. Stages of the mathematical data processing (at Example 1)**

| Variant of the terminal triplet | 1 hour of the DSNP-analysis | | | 2 hours of the DSNP-analysis | | |
|---|---|---|---|---|---|---|
| | The signal level after background subtraction | The signal normalization by multiplication with specific coefficient | Signal percentage | The signal level after background subtraction | The signal normalization by multiplication with specific coefficient | Signal percentage |
| 1.TAG | 5,32 | 5,32 | 17,8 % | 7,34 | 7,34 | 20,4 % |
| 2.AGG | 4,4 | 3,37 | 11,3 % | 5,7 | 3,98 | 11,05 % |
| 3.GGG | 4,44 | 2,72 | 9,1 % | 5,49 | 2,9 | 8,05 % |
| 4.GGT | 6,58 | 1,45 | 4,8 % | 7,16 | 1,27 | 3,5 % |
| 5.GTT | 2,25 | 8,43 | 28,2 % | 3,0 | 9,74 | 27,1 % |
| 6.TTA | 2,91 | 8,63 | 28,8 % | 4,32 | 10,76 | 29,9 % |

4th stage: the percentage averaging of the signals levels received after one and two hours of DSNP analysis. The result of final data processing was the construction of bar diagram displaying an average percentage ratio of the nucleotide endings (terminal triplets) of G-overhangs, and called by the Terminal Nucleotide Profile of the G-strand of telomeric DNA (Fig. 2).

**EXAMPLE 2. Analysis of the G-strand terminal nucleotides of human telomeric DNA extracted from the culture of peripheral blood lymphocytes, stimulated to mitotic division in the presence of phytohemagglutinin (PHA) during 2 hours.**

The stimulation of lymphocytes to mitotic division was made using phytohemagglutinin addition to culture medium RPMI-1640 in final concentration of 10 $\mu$g/ml.

The studying of cyclin gene expression in lymphocyte culture by a method of standard RT-PCR has revealed the sharp growth of mRNA quantity for cyclin D2 against absence of cyclins A2, B1, D3 and E1. It testified to a significant progression of early G1 phase of cell cycle at the considerable part of the cells. The absence of the detected expression of cyclin genes of types A, B and E testified to the absence of mitotic and synthetic phase of the cell cycle. In this connection the examined culture of lymphocytes satisfied to conditions of the nondividing state and of the significant activation of early preparation for cell division.

All analysis stages was made similarly to a procedure described in an Example 1. The consecutive stages of the data processing, received at the duplex-specific analysis, are presented in Table 5.

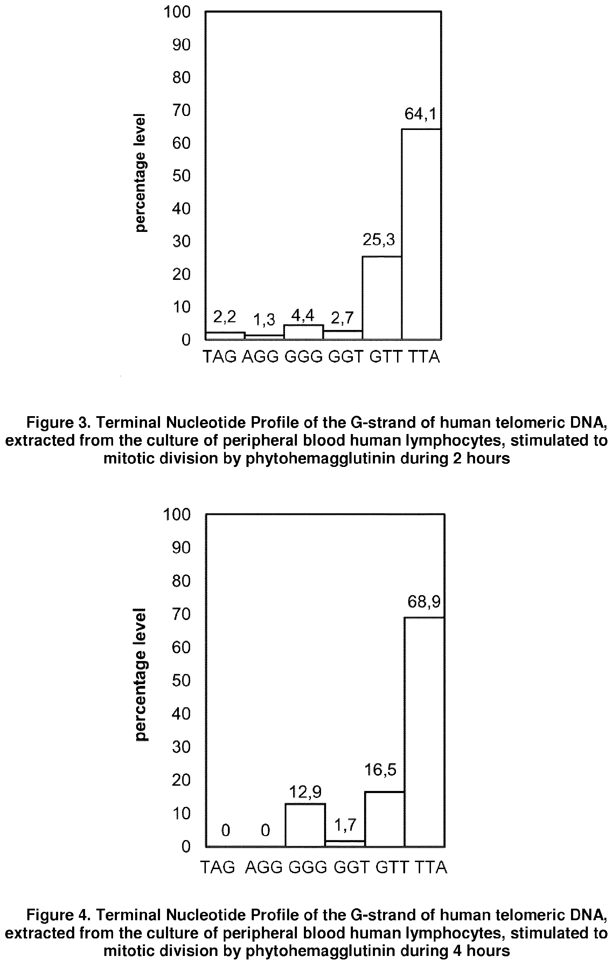

The data processing result is presented by the bar diagram of the average percentage value of fluorescent signals of probes (Fig. 3) and corresponds to a Terminal Nucleotide Profile of the G-strand of telomeric DNA for investigated fraction of lymphocytes, stimulated to mitotic division in the presence of PHA during 2 hours.

**Table 5. Stages of the mathematical data processing (at Example 2)**

| Variant of the terminal triplet | 1 hour of the DSNP-analysis | | | 2 hours of the DSNP-analysis | | |
|---|---|---|---|---|---|---|
| | The signal level after background subtraction | The signal normalization by multiplication with specific coefficient | Signal percentage | The signal level after background subtraction | The signal normalization by multiplication with specific coefficient | Signal percentage |
| 1.TAG | 0,26 | 0,26 | 1,4% | 0,8 | 0,8 | 3,1% |
| 2.AGG | 0,21 | 0,16 | 0,9% | 0,63 | 0,44 | 1,7% |
| 3.GGG | 1,42 | 0,87 | 4,6% | 2,02 | 1,07 | 4,2% |
| 4.GGT | 2,53 | 0,56 | 2,9% | 3,61 | 0,64 | 2,5% |
| 5.GTT | 1,26 | 5,04 | 26,8% | 1,87 | 6,07 | 23,7% |
| 6.TTA | 4,68 | 11,92 | 63,4% | 6,65 | 16,57 | 64,8% |

**EXAMPLE 3. Analysis of the G-strand terminal nucleotides of human telomeric DNA extracted from the culture of peripheral blood lymphocytes, stimulated to mitotic division in the presence of phytohemagglutinin (PHA) during 4 hours.**

The studying of cyclin gene expression in this culture of lymphocytes, like the previous example, has revealed a high mRNA level of cyclin D2 (1,19) against absence of A2, B1, D3 and E1 cyclins. In this connection the examined culture of lymphocytes, as well as culture in previous example, satisfied to conditions of the nondividing state and of the significant activation of early preparation for mitotic division.

All analysis stages was made similarly to a procedure described in an Example 1. The consecutive stages of data processing, received at the duplex-specific analysis, are presented in Table 6.

The data processing result is presented by the bar diagram of the average percentage value of fluorescent signals of probes (Fig. 4) and corresponds to a terminal nucleotides profile of the G-strand of telomeric DNA for investigated fraction of lymphocytes stimulated to mitotic division in the presence of PHA during 4 hours.

**Table 6. Stages of the mathematical data processing (at Example 3)**

| Variant of the terminal triplet | 1 hour of the DSNP-analysis | | | 2 hours of the DSNP-analysis | | |
|---|---|---|---|---|---|---|
| | The signal level after background subtraction | The signal normalization by multiplication with specific coefficient | Signal percentage | The signal level after background subtraction | The signal normalization by multiplication with specific coefficient | Signal percentage |
| 1.TAG | 0 | 0 | 0 % | 0 | 0 | 0 % |
| 2.AGG | 0 | 0 | 0 % | 0 | 0 | 0 % |
| 3.GGG | 3,58 | 2,19 | 13,1 % | 5,58 | 2,95 | 12,6 % |
| 4.GGT | 1,25 | 0,28 | 1,7 % | 2,3 | 0,41 | 1,7 % |
| 5.GTT | 0,68 | 2,55 | 15,3 % | 1,28 | 4,16 | 17,8 % |
| 6.TTA | 3,93 | 11,66 | 69,9 % | 6,38 | 15,9 | 67,9 % |

**EXAMPLE 4. Analysis of the G-strand terminal nucleotides of human telomeric DNA extracted from the culture of peripheral blood lymphocytes, stimulated to mitotic division in the presence of phytohemagglutinin (PHA) during 11 hours.**

The studying of cyclin gene expression in culture of lymphocytes by a method of standard RT-PCR has revealed the presence of all detected cyclins. Following values of cyclin mRNA levels in relation to mRNA of normalizing gene GAPDH were received: A2 (0,25), B1 (0,95), D2 (1,47), D3 (0,91) and E1 (0,17). The high mRNA levels of cyclins D2 and D3 testified to essential progression of G1 phase of the cell cycle at a significant part of cells. At the same time other part of cells was at the synthetic stage of preparation for mitotic division (cyclins E1 and A2) and at the mitosis stage (cyclin B1). Probably, the initial examined culture of lymphocytes was represented the admixed population of cells which were at the different stages of cell cycle. The part of cells even before PHA stimulation already was in G1 and S phases. The further progression of a cell cycle has led to the appearance of synthetic and mitotic phases accordingly after 11 hours of PHA influence.

The investigated culture presents a special interest as allows to define the character of a Terminal Nucleotide Profile existing of the most part of cell cycle. It allows to define, what the profile exists during late G1 phase, synthetic and postsynthetic phases, and also during the mitosis, except for the period of first hours of entering to early G1 phase comprising the trigger mechanism of the beginning of cell division activation.

All analysis stages was made similarly to a procedure described in an Example 1.

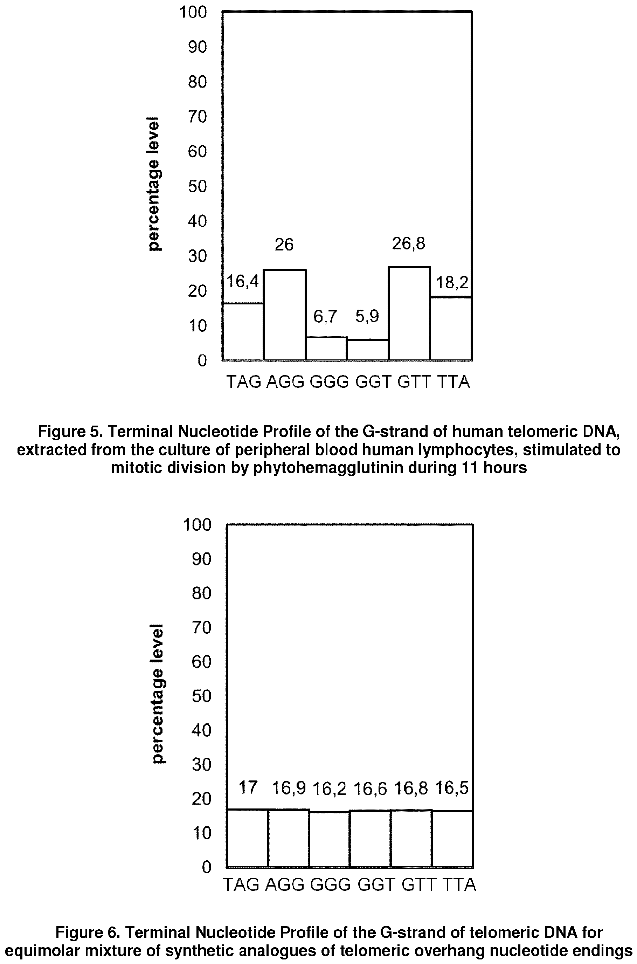

The consecutive stages of data processing received at the duplex-specific analysis are presented in Table 7. The data processing result is presented by plotting of a Terminal Nucleotide Profile of the G-strand of telomeric DNA extracted from the culture of human lymphocytes stimulated to mitotic division in the presence of PHA during 11 hours (Fig. 5).

**Table 7. Stages of the mathematical data processing (at Example 4)**

| Variant of the terminal triplet | 1 hour of the DSNP-analysis | | | 2 hours of the DSNP-analysis | | |
|---|---|---|---|---|---|---|
| | The signal level after background subtraction | The signal normalization by multiplication with specific coefficient | Signal percentage | The signal level after background subtraction | The signal normalization by multiplication with specific coefficient | Signal percentage |
| 1.TAG | 3,32 | 3,32 | 14,9 % | 4,52 | 4,52 | 17,9 % |
| 2.AGG | 7,88 | 6,03 | 27,0 % | 8,9 | 6,33 | 25,0 % |
| 3.GGG | 2,59 | 1,59 | 7,1 % | 3,01 | 1,59 | 6,3 % |
| 4.GGT | 6,19 | 1,37 | 6,1 % | 8,01 | 1,43 | 5,7 % |
| 5.GTT | 1,73 | 6,48 | 29,0 % | 1,91 | 6,20 | 24,6 % |
| 6.TTA | 1,20 | 3,56 | 15,9 % | 2,08 | 5,18 | 20,5 % |

**EXAMPLE 5. Analysis of the G-strand terminal nucleotides of telomeric DNA sample presented by a mixture of synthetic analogues of G-overhang nucleotide endings in equimolar concentration.**

This sample was used as a control for determination of the signal probes concentrations and the coefficients of fluorescent signal normalization at the DSNP-analysis.

In 1,5 ml microtube was prepared a mixture of analyzed telomeric DNA which consisted of 0,5 pmole each of six variants of the G-overhang nucleotide endings. The synthetic analogues of the telomeric overhangs was represented by deoxyribonucleotides in length of 25 nt synthesized in company "Evrogen" (Russia) and terminated on the 3 '-end by triplets: TAG, AGG, GGG, GGT, GTT and TTA. A mixture used for the reaction of polydeoxycytidylation in final volume 40 μl. All following stages of polydeoxyadenylation, synthesis of the negative strands and etc. was made according to procedure described in Example 1.

The figure 1 presents a graphic image of the fluorescence for each probe after one and two hours of DSNP-analysis. The data processing was made according procedure described in an Example 1 using program LabWorks. The consecutive stages of data processing of received fluorescence values for each probe and corresponding nucleotide endings are presented in Table 8. The data processing result is presented by bar diagram of the average percentage value of fluorescent signal growth at the probe cleavage (Fig. 6). The received bar diagram corresponds to the Terminal Nucleotide Profile of the investigated equimolar mixture of synthetic analogues of G-overhang nucleotide endings.

**Table 8. Stages of the mathematical data processing (at Example 5)**

| Variant of the terminal triplet | 1 hour of the DSNP-analysis | | | 2 hours of the DSNP-analysis | | |
|---|---|---|---|---|---|---|
| | The signal level after background subtraction | The signal normalization by multiplication with specific coefficient | Signal percentage | The signal level after background subtraction | The signal normalization by multiplication with specific coefficient | Signal percentage |
| 1.TAG | 5,54 | 5,54 | 17,3 % | 7,86 | 7,86 | 16,8 % |
| 2.AGG | 7,11 | 5,44 | 17,0 % | 11,24 | 7,85 | 16,7 % |
| 3.GGG | 8,33 | 5,1 | 15,9 % | 14,61 | 7,71 | 16,5 % |
| 4.GGT | 23,3 | 5,15 | 16,1 % | 44,94 | 8,0 | 17,1 % |
| 5.GTT | 1,43 | 5,36 | 16,7 % | 2,44 | 7,92 | 16,9 % |
| 6.TTA | 1,84 | 5,46 | 17,0 % | 3,01 | 7,5 | 16,0 % |

Apparently from the bar diagram, the received profile specifies the presence of all variants of the triplet nucleotide endings at an equal interrelation approximately on 16,7% that completely corresponds to the initial equimolar interrelation in a mixture of the synthetic analogues of G-overhang nucleotide endings. The maximum deviation of the received values from theoretically expected compounds no more than 0,5 %. The carrying out of similar researches of the control samples with known quantitative interrelation of telomeric nucleotide endings has displayed the margin of error of the declared method no more than 1 %. This fact allows applying the declared method to obtaining of analysis results with high degree of the confidence.

## INDUSTRIAL APPLICABILITY

The invention can find the application in the fields of public health service and science. The description of invention is enough for realization of the declared method in DNA-diagnostics at the medical and research institutions.

The examples of the invention realization illustrates the possibility of the declared method for definite quantitative assessment of interrelation of the nucleotide endings of telomeric G-strand during a cell cycle but not only for the qualitative test of theirs presence. The declared method suits both for the research of very short telomeric overhangs and of more extended. The efficiency for analysis of the synthetic analogues of telomeric overhangs, as for the natural fractions of G-overhangs presented different lengthy polynucleotides is shown.

In the declared method the full leaving from the ligation procedure led to taken out of dependence of the analysis productivity from the efficiency of ligation and from the length of G-overhang template, like in a method offered Agnel J. Sfeir et al. This fact shows the indisputable technical advantage of the declared method before existing analogues. It allows revealing the changes of the terminal nucleotides of telomeric G-overhangs, descending at the first hours after activation of cell division. The existence and the mutual transitions of equilibrium and nonequilibrium states of Terminal Nucleotide Profile of the G-strand of human telomeric DNA which defines the duration of cell cycle were revealed.

The brightly expressed differences between a Terminal Nucleotide Profile at the 1 st example and profiles at the 2nd and the 3rd examples were received. At the first example the Terminal Nucleotide Profile corresponded to the culture not stimulated to mitotic division by PHA basically belonged to the nondividing state with weak natural activation of early preparation for mitotic division. For this profile the presence of considerable quantity of the nucleotide endings TAG and AGG was characteristically, thus a quantity of TTA terminal triplet was not above 30 %. This Terminal Nucleotide Profile belongs to the particulate balanced on a percentage ratio of telomeric overhang nucleotide endings.

The activation of cell division is accompanied by the formation of unbalanced nonequilibrium profile of the G-strand terminal nucleotides which main feature is the sharp increasing maintenance of terminal triplet TTA. Thus its quantity reaches the level of 60-70 % that was observed in the second and third examples of invention realization, where the cultures of human lymphocytes after 2 and 4 hours of PHA influence were investigated (Fig. 3 and 4).

The analysis of lymphocyte cultures in a resting phase (Go) without visible progression of $G_1$ phase has revealed the presence of the completely balanced equilibrium profile of G-strand terminal nucleotides. The following interrelation of the percentage for the terminal triplets was characteristic for it performance:

$$\textbf{(TAG=TTA=16-20\%) + (GGG=GGT=5-7\%) = (AGG=GTT=25-27\%)}.$$

Thus this profile reminded a figure of "symmetric crown» like a profile, represented on Fig. 5. The comparison such symmetric profile with a profile, received in 1st example, proves the fact that the mechanisms of activation of cell division in the investigated culture of lymphocytes have already been started. The decreasing of AGG variant of the telomeric nucleotide ending as opposed to the moderate growth of TTA variant was testified to it. The further tendency of changes of the Terminal Nucleotide Profile at the activation of mitotic division as a result of PHA stimulant influence during 2 and 4 hours is shown by Examples 2 and 3.

Thereby the decreasing of AGG terminal triplet and the simultaneous increasing of TTA tpiplet is a sign of the beginning of cell proliferation activation and of the condition of normal progression of cell division. During the preparation of cell division under the condition of malignization absence the nonequilibrium profile gradually passes to balanced equilibrium state. This process visually demonstrated by 4th example of the invention realization in which the lymphocyte culture after 11 hours of PHA influence was investigated. Thus the content of TTA variant of the telomeric DNA termini has decreased to 18 % and was approximately equalized with the amount of TAG variant. The content of AGG and GTT variants has compounded approximately in an equal interrelation on 26 % everyone. Apparently from the bar diagram Fig. 5 the received profile completely corresponds to profile of balanced equilibrium state of the terminal nucleotides received for the lymphocyte cultures which were not activated to mitotic division and contained in G0 phase of cell cycle.

For the first time the use of declared method was shown that the initiation of cell division under the influence of any stimulant, for example PHA, is carried out instantly and accompanied by rearrangements of the terminal nucleotides of telomeric DNA. Thus the transition of major nucleotide endings to TTA terminal triplet is the trigger mechanism of the beginning of cell division preparation, specifically, of the transition of G0 phase to early G1 phase.

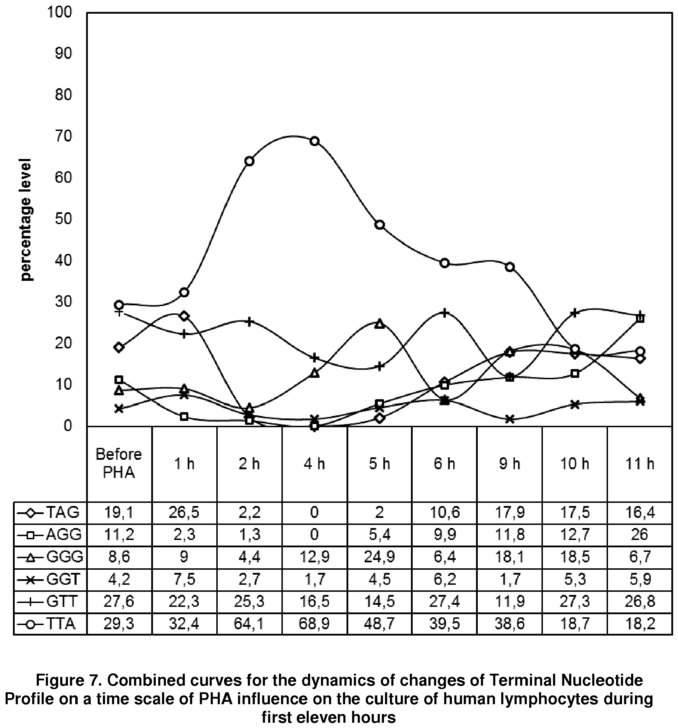

The declared method gives unique possibility for dynamic research of changes of the Terminal Nucleotide Profile due to exact determination of percentage ratio. The possibility of construction of the various graphics (diagrams and curves) displaying the minimal changes of quantitative content of the variants of telomeric overhang nucleotide endings during a cell cycle profitably distinguishes a declared method from all existing analogues. The tracing of the dynamics of changes of Terminal Nucleotide Profile has allowed to determinate their origin. The similar dynamics is represented at Fig. 7 which demonstrates that the curves corresponding to specific variants of the triplet nucleotide endings have wavy character. The quantity changes of concrete variant of telomeric nucleotide ending are related to changes of the next variants. It testifies to the mutual transition of one variant of the overhang nucleotide endings in others as a result of exonuclease influence. Such transitions were clearly traced at the first 11 hours of stimulant influence and carried out in a direction: AGG→TAG→TTA→GTT→GGT→GGG →AGG.

At first two hours the TTA triplet signal promptly increased due to TAG and AGG terminal triplets as a result of consecutive 3'-end single nucleotide removal in a direction: AGG→TAG→TTA. In the same time the content of GTT, GGT and GGG terminal triplets slightly fluctuated due to consecutive transitions in a direction: TTA→GTT→GGT→GGG. The similar transitions of nearby variants of the overhang nucleotide endings in others were displayed throughout whole graphics on Fig. 7. Thus their quantitative interrelations were observed exactly. If the level of one terminal triplet was decreased the level of next triplet with transition in one-two nucleotides was increased. The existing mechanism of the consecutive 3'-end single nucleotide transitions of telomeric G-overhang nucleotide endings is original molecular mechanism which was named «the Nucleotide Clock». It provides the trigger mechanism of cell division activation on one hand and defines the existence of the balanced equilibrium profile of terminal nucleotides during larger time of the cell cycle on other hand.

The existence of the balanced equilibrium profile of terminal nucleotides during a larger part of cell cycle is the protective molecular mechanism of healthy cells. It prevents the premature beginning of new cell division and defines the duration of cell cycle. For the healthy peripheral blood lymphocytes, stimulated to mitotic division by PHA, it compounds approximately 48 hours. The following unbalanced nonequilibrium profile cannot arise earlier than through the positioned number of hours after the beginning of initial influence of the stimulating agent. The approach of the balanced equilibrium state of TNP, since 10-12 hours after the beginning of mitotic division stimulation, and its maintenance during a larger part of the cell cycle are an important diagnostic sign of healthy cells.

The cell cycle length reduction due to earlier beginning of a following nonequilibrium state of TNP and its existence during more long time than phase G1 of the cell cycle is the characteristic feature of cell malignization. Thus the disorders of balanced equilibrium profile formation related with blockage of the cycle of consecutive single nucleotide transitions on the 3'-end of telomeric G-strand can lead to long prevalence of TTA variant of its ending. Possibly the TTA terminal triplet of the G-strand of telomeric DNA being on the end of concrete chromosomes is responsible for the activation of gene expression which defining the cell division stimulation and the specific phenotypic variation of cancer cells. The conditions of constant activation of cell division and the cell cycle length reduction due to long prevalence of TTA nucleotide ending of telomeric DNA can be the main reason of cancer.

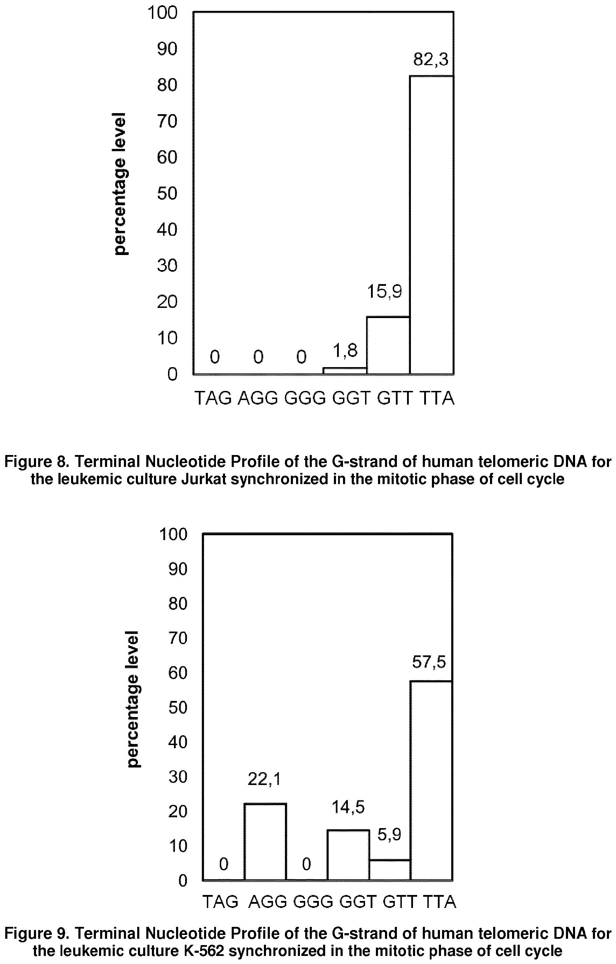

The researches of the leukemic cultures Jurkat and K-562 of human lymphocytes, synchronized in a mitotic phase of cell cycle was made using the declared method. The synchronization of cells was carried out at the colchicine addition to the culture medium RPMI-1640 in final concentration of 0,5 $\mu$g/ml during 12 hours. The long duration of colchicine influence has provided the maximum accumulation of cells in the mitotic phase. The synchronization of the leukemic cells in a mitotic metaphase has revealed the absence of balanced equilibrium profile of terminal nucleotides. Thus degree of the balance disorders of Terminal Nucleotide Profile correlated with the degree of cell malignization and with their mitotic division rate. The Jurkat culture which had twice by larger rate of the mitotic division in comparison with other investigated culture was characterized by the extremely unbalanced TNP (Fig. 8). This profile was mainly presented by the TTA nucleotide ending (82,3 %) and very insignificant presence of the GTT (15,9 %) and GGT (1,8 %) terminal triplets. The absence of other variants of telomeric nucleotide endings, especially AGG and TAG triplets, testified to the blockage of the cycle of consecutive single nucleotide transitions on the 3'-end of telomeric DNA. The particulate blockage of transitions for the terminal triplets TTA→GTT→GGT and full blockage of transitions for the triplets GGT→GGG→AGG→TAG was the result of absence of the balanced equilibrium profile of terminal nucleotides.

The K-562 culture also was characterized by the presence of unbalanced profile of terminal nucleotides, but with smaller degree of the disbalance (Fig.9). Against the prevalence of TTA terminal triplet (57,5 %) there were the triplets AGG (22,1 %), GGT (14,5 %) and GTT (5,9 %) which testified to depression of the terminal nucleotide transitions cycle of telomeric nucleotide endings leading to formation of their balanced equilibrium profile. The characteristic feature of the both lymphocytic cultures was the absence of TAG and GGG variants of telomeric DNA termini. Probably for the terminal triplet TAG it is bound to its full transition in TTA triplet as the most close single nucleotide transition. For culture Jurkat all of the terminal triplets GGG, AGG and TAG were transited to the TTA terminal triplet that lead to its very high percentage content (82,3%) which correlated with very high rate of the mitotic division.

Our studies have shown that the terminal triplet TTA is a marker of cell division activation relates to the entering to G1 phase. The terminal triplet AGG is a marker of nondividing state which has high percentage content (25-27%) at the cells in G0 phase excepting of the trigger moment of division activation at the entering to G1 phase. The terminal triplet GGG is a marker of cell senescence and of the depletion state of cell medium. It had an increased percentage content (up from 5-7% to 18-20%) at the cells in adverse conditions due to decrease of the AGG triplet content (down from 25-27% to 12-13%). So the reduction or absence of the GGG and AGG variants of telomeric nucleotide endings is the main feature of cancer cells.

Accordingly the determination of presence or absence of the balanced equilibrium profile of terminal nucleotides and also the degree of its disbalance at cell synchronization in specific to it phases of the cell cycle (for example, in mitotic phase), is the important diagnostic criterion in oncology. The studying of TNP changes during a cell cycle of cancer cells, the determinations of the duration of nonequilibrium profile existence and the disorders of formation of its equilibrium state can be a criterion defining the degree of cell malignization.

The invention allows to catch the minimum changes in the percentage of each variants of telomeric nucleotide endings and can serve as the universal research instrument of the cell cycle of any cells and tissues. The Terminal Nucleotide Profiles of the G-strand of telomeric DNA can be composed for definition of the proliferative status of various cells and tissues in diagnostic and medical institutions, in scientific research laboratories. The plotting of percentage profiles of the terminal nucleotides of telomeric DNA for immune cells can reveal the degree of their activation for mitotic division and be the criterion of immune status of human organism. The use of the declared method for research of proliferative potential of the stem cells and any cells capable to cell division can find the application in transplantology as the criterion of cell adaptation. In cosmetology, dermatology and gerontology it can use like the factor of regenerative abilities of tissues and the organism potential to a rejuvenation and as the criterion of definition of the human biological age.

## Claims

1. A method for the quantitative analysis of the G-strand terminal nucleotides of human telomeric DNA comprising the following stages (a)-(d):

(a) extraction of the genomic DNA and the subsequent isolation of the 3'-overhanging single-stranded ends of telomeric DNA (G-overhangs) comprising the steps (i) and (ii):

(i) cleavage of the double-stranded genomic DNA which preserves the single-stranded telomeric G-overhangs with the aid of duplex-specific nuclease enzyme;
(ii) isolation of the telomeric G-overhangs by means of the magnetic beads that have complementary oligonucleotide probes immobilized on the surface thereof;

(b) amplification of the telomeric G-overhangs comprising the steps (i)-(iv):

(i) 3'-modification by attachment of a polydeoxycytidylic sequence and a polydeoxyadenylic sequence situated downstream thereof with the aid of terminal deoxynucleotidyl transferase enzyme;
(ii) amplification using a primer having SEQ ID NO: 1 in order to obtain negative strands containing an adapter sequence having SEQ ID NO: 3;
(iii) 3'-modification of the negative strands by attachment of a polydeoxyadenylic sequence with the aid of terminal deoxynucleotidyl transferase enzyme;
(iv) final asymmetric PCR amplification of the negative strands using a pair of primers set out in SEQ ID NO: 3 and SEQ ID NO: 4 under an excess of adapter sequence having SEQ ID NO: 3;

(c) duplex-specific analysis of the G-overhang negative strands comprising the steps (i) and (ii):

(i) hybridization with the signal oligonucleotide probes which are fluorescent-labeled with dye FAM and quencher RTQ1 and correspond to six possible nucleotide triplet endings of the G-strand of human telomeric DNA and are set out in SEQ ID NOs: 6-11;
(ii) cleavage of the perfect DNA duplexes in the presence of duplex-specific nuclease enzyme leading to increase of the fluorescent signals of probes which are specific to detectable telomeric overhang endings;

(d) recording the results of the duplex-specific analysis comprising the steps (i)-(iii):

(i) measuring the fluorescence levels of the signal probes during hydrolysis in the presence of duplex-specific nuclease enzyme with the aid of fluorescence detection device;
(ii) mathematical processing the data by normalizing the fluorescent signals by means of subtraction of the background before hydrolysis and multiplication by the specific coefficients calculated for each probe in measuring intervals of the analysis;
(iii) final assessment of the quantitative relationship of fluorescence levels of the signal probes corresponding to possible G-overhang nucleotide endings by plotting the Terminal Nucleotide Profile of the G-strand of telomeric DNA in percent.

2. The method as claimed in claim 1, wherein any hybridization or sorption methods using membranes, columns, beads or other surfaces are used for immobilization and isolation of telomeric G-overhangs in step (a)(ii).

3. A method for the quantitative analysis of the G-strand terminal nucleotides of human telomeric DNA comprising the following stages (a)-(d):

(a) extraction of the genomic DNA and the subsequent isolation of the 3'-overhanging single-stranded ends of telomeric DNA (G-overhangs) comprising the steps (i) and (ii):

(i) cleavage of the double-stranded genomic DNA which preserves the single-stranded telomeric G-overhangs with the aid of duplex-specific nuclease enzyme;
(ii) isolation of the telomeric G-overhangs by means of the magnetic beads that have complementary oligonucleotide probes immobilized on the surface thereof;

(b) amplification of the telomeric G-overhangs comprising the step (i)-(iv):

(i) attachment of polydeoxythymidylic sequence after polydeoxycytidylic sequence with the aid of terminal deoxynucleotidyl transferase enzyme,
(ii) amplification of the negative strands using a primer having SEQ ID NO: 2 in order to obtain negative strands containing an adapter sequence having SEQ ID NO: 3;
(iii) attachment of polydeoxythymidylic sequence to negative strands using terminal deoxynucleotidyl transferase enzyme;
(iv) final asymmetric PCR amplification using a pair of primers set out in SEQ ID NO: 3 and SEQ ID NO: 5

under an excess of adapter sequence having SEQ ID NO: 3;

(c) duplex-specific analysis of the G-overhang negative strands comprising the steps (i) and (ii):

(i) hybridization with the signal oligonucleotide probes which are fluorescent-labeled with dye FAM and quencher RTQ1 and correspond to six possible nucleotide triplet endings of the G-strand of human telomeric DNA and are set out in SEQ ID NOs: 6-11;
(ii) cleavage of the perfect DNA duplexes in the presence of duplex-specific nuclease enzyme leading to increase of the fluorescent signals of probes which are specific to detectable telomeric overhang endings;

(d) recording the results of the duplex-specific analysis comprising the steps (i)-(iii):

(i) measuring the fluorescence levels of the signal probes during hydrolysis in the presence of duplex-specific nuclease enzyme with the aid of fluorescence detection device;
(ii) mathematical processing the data by normalizing the fluorescent signals by means of subtraction of the background before hydrolysis and multiplication by the specific coefficients calculated for each probe in measuring intervals of the analysis;
(iii) final assessment of the quantitative relationship of fluorescence levels of the signal probes corresponding to possible G-overhang nucleotide endings by plotting the Terminal Nucleotide Profile of the G-strand of telomeric DNA in percent.

4. The method as claimed in claim 1 or claim 3, wherein at the amplification of the telomeric G-overhangs at stage (b) any oligonucleotide sequence which is not complementary to repeats of the G-strand of human telomeric DNA is used instead of the adapter sequence presented in SEQ ID NOs: 1-3.

5. The method as claimed in claim 1, wherein oligonucleotide variants of the signal probes having a length of 10-12 nucleotides are used at the duplex-specific analysis at the stage (c) instead of the signal probes presented in SEQ ID NOs: 6-11.

6. The method as claimed in claim 1, wherein the signal oligonucleotide probes used in step (c), instead of being fluorescent-labeled with dye FAM and quencher RTQ1, are labeled on opposite ends with a combination "dye-quencher" comprising any fluorescent dye, including FITC, FAM, R110, TET, R6G, HEX, JOE, VIC, Yakima Yellow, TAMRA, Cy3, ROX, Super ROX, Cy 3.5, Cy5, Cy 5.5, Cy7, Cy 7.5, quenchers RTQ, BHQ, Dabcyl and others.

7. The method as claimed in claim 1, wherein any kinds of the fluorescence detection devices and software allowing to quantitatively measure the levels of a significant fluorescent signal for each probe are used for the recording the results of the duplex-specific analysis at stage (d), including the multichannel registration of different kinds of labeling in claim 6.

8. The method as claimed in claim 1, wherein the mathematical processing of the fluorescent signals at stage (d)(ii) consists in normalizing the signals by means of multiplication by specific coefficients presented in the Table 1 for each FAM-RTQ1 probe during measuring intervals of 1 and 2 hours of the analysis.

9. The method as claimed in claim 1, wherein the mathematical processing of the fluorescent signals at stage (d)(ii) consists in normalizing the signals by means of multiplication by specific coefficients distinct from the Table 1 and calculated for different kinds of fluorescent labeling according claim 6, various concentrations of the probes and which are corresponding to different measuring intervals of the analysis and current moment of the research.

10. The method as claimed in claim 1, wherein the results of analysis at the stage (d)(iii) consisting in plotting of Terminal Nucleotide Profiles of the G-strand of human telomeric DNA are used such as defining criterion in medical diagnostics of the diseases and ageing of human organism, including an assessment of proliferative properties and tissue-specific functions of cells, the definition of prognosis of their development and a choice of the specific therapy.

11. A set of the specific deoxyribo-oligonucleotides presented in SEQ ID NOs: 1-5 for amplification of the negative strands of telomeric G-overhangs during PCR in a method according to claim 1.

12. The set of the specific deoxyribo-oligonucleotides as claimed in claim 11 comprising the any oligonucleotide sequence not complementary to repeats of the G-strand of human telomeric DNA instead of the adapter sequence presented in SEQ ID NOs: 1-3.

13. A set of the specific deoxyribo-oligonucleotides presented in SEQ ID NOs: 6-11 for fluorescent labeling of signal probes at the duplex-specific analysis in a method according to claim 1.

14. The set of the specific deoxyribo-oligonucleotides as claimed in claim 13 comprising the different kinds of the fluorescent probe labeling with combination "dye-quencher" on any opposite ends of the probe consisting of any fluorescent dyes, including FITC, FAM, R110, TET, R6G, HEX, JOE, VIC, Yakima Yellow, TAMRA, Cy3, ROX, Super ROX, Cy 3.5, Cy5, Cy 5.5, Cy7, Cy 7.5, quenchers RTQ, BHQ, Dabcyl and others.

**Patentansprüche**

1. Verfahren zur quantitativen Analyse der terminalen G-Strang-Nukleotide der menschlichen Telomer-DNA, umfassend die folgenden Phasen (a) - (d):

(a) Extraktion der genomen DNA und die anschließende Isolierung der 3'-überhängenden einzelsträngigen Enden der Telomer-DNA (G-Überhänge), umfassend die Schritte (i) und (ii):

(i) Spalten der doppelsträngigen genomen DNA, welche die einzelsträngigen Telomer-G-Überhänge mithilfe des duplexspezifischen Nukleaseenzyms konserviert;
(ii) Isolieren der Telomer-G-Überhänge durch die magnetischen Kügelchen, die zu komplementären Oligonukleotidsonden geworden sind, die an deren Oberfläche immobilisiert sind;

(b) Verstärkung der Telomer-G-Überhänge, umfassend die Schritte (i) - (iv):

(i) 3'-Modifikation durch Anbindung einer Polydeoxycytidylsequenz und eine Polydeoxyadenylsequenz, die sich dieser nachgeordnet befindet, mithilfe des terminalen Deoxynukleotidyl-Transferaseenzyms;
(ii) Verstärken unter Verwendung eines Primers mit der SEQ ID NO: 1, um negative Stränge zu erhalten, die eine Adaptersequenz mit der SEQ ID NO: 3 enthalten;
(iii) 3'-Modifikation der negativen Stränge durch Anbinden einer Polydeoxyadenylsequenz mithilfe des Deoxynukleotidyl-Transferaseenzyms;
(iv) endgültiges asymmetrisches PCR-Verstärken der negativen Stränge unter Verwendung eines Paars von Primern, wie in SEQ ID NO: 3 und SEQ ID NO: 4 unter einem Überschuss der Adaptersequenz mit der SEQ ID NO: 3 festgelegt;

(c) duplexspezifische Analyse der negativen G-Überhangstränge, umfassend die Schritte (i) und (ii):

(i) Hybridisieren mit den Signal-Oligonukleotidsonden, die mit dem Farbstoff FAM und dem Quencher RTQ1 fluoreszent markiert sind und sechs möglichen Nukleotid-Dreifachendungen des G-Strangs von menschlicher Telomer-DNA markiert sind und in den SEQ ID NO: 6 - 11 festgelegt sind;
(ii) Spalten der perfekten DNA-Duplexe in Anwesenheit des duplexspezifischen Nukleaseenzyms, was zu einer Erhöhung des fluoreszenten Signals der Sonden führt, die spezifisch für erfassbare Telomerüberhangendungen sind;

(d) Aufzeichnen der Ergebnisse der duplexspezifischen Analyse, umfassend die Schritte (i) - (iii):

(i) Messen der Fluoreszenzwerte der Signalsonden während der Hydrolyse in Anwesenheit des duplexspezifischen Nukleaseenzyms mithilfe der Fluoreszenz-Erfassungsvorrichtung;
(ii) mathematische Verarbeitung der Daten durch Normalisieren des fluoreszenten Signals mittels Subtraktion des Hintergrunds vor der Hydrolyse und Multiplikation durch spezifische Koeffizienten, die beim Messen von Intervallen der Analyse für jede Sonde berechnet werden;
(iii) endgültiges Bewerten des quantitativen Verhältnisses der Fluoreszenzwerte der Signalsonden, die möglichen G-Überhang-Nukleotidenden entsprechen, durch grafische Darstellung des terminalen Nukleotidprofils des G-Strangs der Telomer-DNA in Prozent.

2. Verfahren nach Anspruch 1, wobei beliebige Hybridisierungs- oder Sorptionsverfahren, die Membrane, Säulen, Kügelchen oder andere Oberflächen einsetzen, für die Immobilisierung und Isolierung der Telomer-G-Überhänge in Schritt (a) (ii) verwendet werden.

3. Verfahren zur quantitativen Analyse der terminalen G-Strang-Nukleotide der menschlichen Telomer-DNA, umfassend die folgenden Phasen (a) - (d):

(a) Extraktion der genomen DNA und die anschließende Isolierung der 3'-überhängenden einzelsträngigen Enden der Telomer-DNA (G-Überhänge), umfassend die Schritte (i) und (ii):

(i) Spalten der doppelsträngigen genomen DNA, welche die einzelsträngigen Telomer-G-Überhänge mithilfe des duplexspezifischen Nukleaseenzyms konserviert;
(ii) Isolieren der Telomer-G-Überhänge durch die magnetischen Kügelchen, die zu komplementären Oligonukleotidsonden geworden sind, die an deren Oberfläche immobilisiert sind;

(b) Verstärkung der Telomer-G-Überhänge, umfassend die Schritte (i) - (iv):

(i) Anbinden der Polydeoxythymidylsequenz nach der Polydeoxycytidylsequenz mithilfe des terminalen Deoxynukleotidyl-Transferaseenzyms,
(ii) Verstärken der negativen Stränge unter Verwendung eines Primers mit der SEQ ID NO: 2, um negative Stränge zu erhalten, die eine Adaptersequenz mit der SEQ ID NO: 3 enthalten;
(iii) Anbinden der Polydeoxythymidylsequenz an negative Stränge unter Verwendung des terminalen Deoxynukleotidyl-Transferaseenzyms;
(iv) endgültiges asymmetrisches PCR-Verstärken unter Verwendung eines Paars von Primern, wie in SEQ ID NO: 3 und SEQ ID NO: 5 unter einem Überschuss der Adaptersequenz mit der SEQ ID NO: 3 festgelegt;

(c) duplexspezifische Analyse der negativen G-Überhangstränge, umfassend die Schritte (i) und (ii):

(i) Hybridisieren mit den Signal-Oligonukleotidsonden, die mit dem Farbstoff FAM und dem Quencher RTQ1 fluoreszent markiert sind und sechs möglichen Nukleotid-Dreifachendungen des G-Strangs von menschlicher Telomer-DNA markiert sind und in den SEQ ID NO: 6 - 11 festgelegt sind;
(ii) Spalten der perfekten DNA-Duplexe in Anwesenheit des duplexspezifischen Nukleaseenzyms, was zu einer Erhöhung des fluoreszenten Signals der Sonden führt, die spezifisch für erfassbare Telomerüberhangendungen sind;

(d) Aufzeichnen der Ergebnisse der duplexspezifischen Analyse, umfassend die Schritte (i) - (iii):

(i) Messen der Fluoreszenzwerte der Signalsonden während der Hydrolyse in Anwesenheit des duplexspezifischen Nukleaseenzyms mithilfe der Fluoreszenz-Erfassungsvorrichtung;
(ii) mathematische Verarbeitung der Daten durch Normalisieren des fluoreszenten Signals mittels Subtraktion des Hintergrunds vor der Hydrolyse und Multiplikation durch spezifische Koeffizienten, die beim Messen von Intervallen der Analyse für jede Sonde berechnet werden;
(iii) endgültiges Bewerten des quantitativen Verhältnisses der Fluoreszenzwerte der Signalsonden, die möglichen G-Überhang-Nukleotidenden entsprechen, durch grafische Darstellung des terminalen Nukleotidprofils des G-Strangs der Telomer-DNA in Prozent.

4. Verfahren nach Anspruch 1 oder Anspruch 3, wobei bei der Verstärkung der Telomer-G-Überhänge in Phase (b) eine beliebige Oligonukleotidsequenz, die nicht komplementär zu den Wiederholungen des G-Strangs der menschlichen Telomer-DNA ist, verwendet wird, anstelle der Adaptersequenz, die in SEQ ID NO: 1 - 3 repräsentiert ist.

5. Verfahren nach Anspruch 1, wobei die Oligonukleotidvarianten der Signalsonden mit einer Länge von 10 - 12 Nukleotiden in der duplexspezifischen Analyse in der Phase (c) anstelle der Signalsonden verwendet werden, die in SEQ ID NO: 6 - 11 repräsentiert sind.

6. Verfahren nach Anspruch 1, wobei die im Schritt (c) verwendeten Oligonukleotidsignalsonden, anstatt mit dem Farbstoff FAM und dem Quencher RTQ1 fluoreszenzmarkiert zu sein, an entgegengesetzten Enden mit einem Kombinations-"Farbstoff-Quencher" markiert sind, umfassend einschließlich FITC, FAM, R110, TET, R6G, HEX, JOE, VIC, Yakima-Gelb, TAMRA, Cy3, ROX, Super ROX, Cy 3.5, Cy5, Cy 5.5, Cy7, Cy 7.5, Quencher RTQ, BHQ, Dabcyl und andere.

7. Verfahren nach Anspruch 1, wobei beliebige Arten von Fluoreszenz-Erfassungsvorrichtungen und Software, die das quantitative Messen der Werte eines signifikanten fluoreszenten Signals für jede Sonde erlauben, zum Aufzeichnen der Ergebnisse der duplexspezifischen Analyse in Phase (d) verwendet werden, einschließlich der Mehrkanalregistrierung verschiedener Arten von Markierungen in Anspruch 6.

8. Verfahren nach Anspruch 1, wobei die mathematische Verarbeitung des fluoreszenten Signals in Phase (d) (ii) aus dem Normalisieren der Signale durch Multiplikation mit spezifischen Koeffizienten, die in der Tabelle 1 für jede FAM-RTQ1-Sonde während dem Messen der Intervalle von 1 und 2 Stunden der Analyse dargelegt sind.

9. Verfahren nach Anspruch 1, wobei die mathematische Verarbeitung der fluoreszenten Signale in Phase (d) (ii) aus dem Normalisieren der Signale durch Multiplikation mit spezifischen Koeffizienten besteht, die sich von der Tabelle 1 unterscheiden, und für verschiedene Arten von fluoreszenter Markierung gemäß Anspruch 6, verschiedene Konzentrationen der Sonden, die den unterschiedlichen Messungsintervallen der Analyse und dem derzeitigen Stand der Forschung berechnet werden.

10. Verfahren nach Anspruch 1, wobei die Ergebnisse der Analyse in der Phase (d) (iii) aus der grafischen Darstellung des terminalen Nukleotidprofils des G-Strangs der verwendeten Telomer-DNA bestehen, wie z.B. dem Definieren der Kriterien der medizinischen Diagnostiken der Krankheiten und der Altersbestimmung des menschlichen Organismus, einschließlich einer Bewertung der proliferativen Merkmale und gewebespezifischen Funktionen von Zellen, der Definition der Prognose und ihre Entwicklung sowie einer Auswahl der spezifischen Therapie.

11. Satz der spezifischen Deoxyribo-Oligonukleotide, die in SEQU ID NO: 1 - 5 zum Verstärken der negativen Stränge der Telomer-G-Überhänge während der PCR bei einem Verfahren gemäß Anspruch 1 repräsentiert sind.

12. Satz der spezifischen Deoxyribo-Oligonukleotide nach Anspruch 11, umfassend die beliebige Oligonukleotidsequenz, nicht nicht komplementär zu den Wiederholungen des G-Strangs von menschlicher Telomer-DNA anstelle der in SEQ ID NO: 1 - 3 repräsentierten Adaptersequenz ist.

13. Satz der spezifischen Deoxyribo-Oligonukleotide, die in SEQ ID NO: 6 - 11 zum fluoreszenten Markieren von Signalsonden in der duplexspezifischen Analyse bei einem Verfahren gemäß Anspruch 1 repräsentiert sind.

14. Satz der spezifischen Deoxyribo-Oligonukleotide nach Anspruch 13, umfassend die verschiedenen Arten der fluoreszenten Sondenmarkierung mit einem Kombinations-"Farbstoff-Quencher" an beliebigen entgegengesetzten Enden der Sonde, bestehend aus beliebigen fluoreszenten Farbstoffen, einschließlich FITC, FAM, R110, TET, R6G, HEX, JOE, VIC, Yakima-Gelb, TAMRA, Cy3, ROX, Super ROX, Cy 3.5, Cy5, Cy 5.5, Cy7, Cy 7.5, Quencher RTQ, BHQ, Dabcyl und anderen.

**Revendications**

1. Procédé pour l'analyse quantitative des nucléotides terminaux du brin G d'un ADN télomérique humain comprenant les étapes (a)-(d) suivantes :

(a) l'extraction de 1'ADN génomique et l'isolement ultérieur des extrémités simple brin en porte-à-faux 3' de l'ADN télomérique (porte-à-faux G) comprenant les étapes (i) et (ii) :

(i) un clivage de l'ADN génomique double brin qui préserve les porte-à-faux G télomériques simple brin à l'aide d'une enzyme nucléase duplex-spécifique ;
(ii) un isolement des porte-à-faux G télomériques au moyen de billes magnétiques qui possèdent des sondes oligonucléotidiques complémentaires immobilisées sur leur surface ;

(b) l'amplification des porte-à-faux G télomériques comprenant les étapes (i)-(iv) :

(i) une modification en 3' par l'attachement d'une séquence polydésoxycytidylique et d'une séquence polydésoxyadénylique située en aval de celle-ci à l'aide d'une enzyme désoxynucléotidyl-transférase terminale ;
(ii) une amplification en utilisant une amorce de SEQ ID NO: 1 afin d'obtenir des brins négatifs contenant une séquence d'adaptateur de SEQ ID NO: 3 ;

(iii) une modification en 3' des brins négatifs par l'attachement d'une séquence polydésoxyadénylique á l'aide d'une enzyme désoxynucleotidyl-transférase terminale ;

(iv) une amplification finale par PCR asymétrique des brins négatifs en utilisant une paire d'amorces décrite dans SEQ ID NO: 3 et SEQ ID NO: 4 dans un excès de séquence d'adaptateur de SEQ ID NO: 3 ;

(c) une analyse duplex-spécifique des brins négatifs de porte-á-faux G comprenant les étapes (i) et (ii) :

(i) une hybridation avec des sondes oligonucléotidiques signal qui sont marquées par fluorescence avec un colorant FAM et un désactivateur RTQ1 et correspondent à six terminaisons de triplets de nucléotides possibles du brin G d'un ADN télomérique humain et sont décrites dans SEQ ID NO: 6-11 ;

(ii) un clivage des duplex d'ADN parfaits en présence d'une enzyme nucléase duplex-spécifique entraînant une augmentation des signaux fluorescents des sondes qui sont spécifiques aux terminaisons de porte-à-faux télomériques détectables ;

(d) l'enregistrement des résultats de l'analyse duplex-spécifique comprenant les étapes (i)-(iii) ;

(i) une mesure des niveaux de fluorescence des sondes signal pendant une hydrolyse en présence d'une enzyme nucléase duplex-spécifique à l'aide d'un dispositif de détection de fluorescence ;

(ii) un traitement mathématique des données en normalisant les signaux fluorescents au moyen d'une soustraction du bruit de fond avant l'hydrolyse et d'une multiplication par les coefficients spécifiques calculées pour chaque sonde dans les intervalles de mesure de l'analyse ;

(iii) une évaluation finale de la relation quantitative des niveaux de fluorescence des sondes signal correspondant à des terminaisons de nucléotides de porte-à-faux G possibles par une représentation graphique du profil des nucléotides terminaux du brin G de l'ADN télomérique en pourcentage.

2. Procédé selon la revendication 1, dans lequel quelconques procédés d'hybridation ou de sorption utilisant des membranes, des colonnes, des billes ou d'autres surfaces, sont utilisés pour l'immobilisation et l'isolement de porte-à-faux G télomériques à l'etape (a)(ii).

3. Procédé pour l'analyse quantitative des nucléotides terminaux du brin G d'un ADN télomérique humain comprenant les étapes (a)-(d) suivantes :

(a) l'extraction de l'ADN génomique et l'isolement ultérieur des extrémités simple brin en porte-à-faux 3' de l'ADN télomérique (porte-à-faux G) comprenant les étapes (i) et (ii) :

(i) un clivage de l'ADN génomique double brin qui préserve les porte-à-faux G télomériques simple brin à l'aide d'une enzyme nucléase duplex-spécifique ;

(ii) un isolement des porte-à-faux G télomériques au moyen de billes magnétiques qui possèdent des sondes oligonucléotidiques complémentaires immobilisées sur leur surface ;

(b) l'amplification des porte-à-faux G télomériques comprenant les étapes (i)-(iv) :

(i) l'attachement d'une séquence polydésoxythymidylique après une séquence polydésoxycytidylique à l'aide d'une enzyme désoxynucléotidyl-transférase terminale,

(ii) l'amplification des brins négatifs en utilisant une amorce de SEQ ID NO: 2 afin d'obtenir des brins négatifs contenant une séquence d'adaptateur de SEQ ID NO: 3 ;

(iii) l'attachement d'une séquence polydésoxythymidylique aux brins négatifs en utilisant une enzyme désoxynucléotidyl-transférase terminale ;

(iv) une amplification finale par PCR asymétrique en utilisant une paire d'amorces décrite dans SEQ ID NO: 3 et SEQ ID NO: 5 dans un excès de séquence d'adaptateur de SEQ ID NO: 3 ;

(c) une analyse duplex-spécifique des brins négatifs de porte-á-faux G comprenant les étapes (i) et (ii) :

(i) une hybridation avec des sondes oligonucléotidiques signal qui sont marquées par fluorescence avec un colorant FAM et un désactivateur RTQ1 et correspondent à six terminaisons de triplets de nucléotides possibles du brin G d'un ADN télomérique humain et sont décrites dans SEQ ID NO: 6-11 ;

(ii) un clivage des duplex d'ADN parfaits en présence d'une enzyme nucléase duplex-spécifique entraînant une augmentation des signaux fluorescents des sondes qui sont spécifiques aux terminaisons de porte-à-faux télomériques détectables ;

(d) l'enregistrement des résultats de l'analyse duplex-spécifique comprenant les étapes (i)-(iii) ;

(i) une mesure des niveaux de fluorescence des sondes signal pendant une hydrolyse en présence d'une enzyme nucléase duplex-spécifique à l'aide d'un dispositif de détection de fluorescence ;
(ii) un traitement mathématique des données en normalisant les signaux fluorescents au moyen d'une soustraction du bruit de fond avant l'hydrolyse et d'une multiplication par les coefficients spécifiques calculées pour chaque sonde dans les intervalles de mesure de l'analyse ;
(iii) une évaluation finale de la relation quantitative des niveaux de fluorescence des sondes signal correspondant à des terminaisons de nucléotides de porte-à-faux G possibles par une représentation graphique du profil des nucléotides terminaux du brin G de l'ADN télomérique en pourcentage.

4. Procédé selon la revendication 1 ou la revendication 3, dans lequel, lors de l'amplification des porte-à-faux G télomériques à l'étape (b), quelconque séquence oligonucléotidique qui n'est pas complémentaire à des répétitions du brin G de l'ADN télomérique humain est utilisée à la place de la séquence d'adaptateur présentée dans SEQ ID NO: 1-3.

5. Procédé selon la revendication 1, dans lequel des variants oligonucléotidiques des sondes signal d'une longueur de 10-12 nucléotides sont utilisées lors de l'analyse duplex-spécifique à l'étape (c) à la place des sondes signal présentées dans SEQ ID NO: 6-11.

6. Procédé selon la revendication 1, dans lequel les sondes signal oligonucléotidiques utilisées dans l'étape (c), au lieu d'être marquées par fluorescence avec colorant FAM et désactivateur RTQ1, sont marquées sur les extrémités opposées avec une combinaison « colorant-désactivateur » comprenant le FITC, FAM, R110, TET, R6G, HEX, JOE, VIC, Yakima Yellow, TAMRA, Cy3, ROX, Super ROX, Cy 3.5, Cy5, Cy 5.5, Cy7, Cy 7.5, les désactivateurs RTQ, BHQ, le Dabcyl et d'autres.

7. Procédé selon la revendication 1, dans lequel de quelconques types de dispositifs de détection de fluorescence et logiciel permettant de mesurer de manière quantitative les niveaux d'un signal fluorescent significatif pour chaque sonde sont utilisés pour l'enregistrement des résultats de l'analyse duplex-spécifique à l'étape (d), comprenant un enregistrement multicanal des différents types de marquage dans la revendication 6.

8. Procédé selon la revendication 1, dans lequel le traitement mathématique des signaux fluorescents à l'étape (d) (ii) consiste en une normalisation des signaux au moyen d'une multiplication par des coefficients spécifiques présentés dans le Tableau 1 pour chaque sonde FAM-RTQ1 pendant les intervalles de mesure de 1 et 2 heures de l'analyse.

9. Procédé selon la revendication 1, dans lequel le traitement mathématique des signaux fluorescents à l'étape (d) (ii) consiste en une normalisation des signaux au moyen d'une multiplication par des coefficients spécifiques distincts du Tableau 1 et calculés pour différents types de marquage fluorescent selon la revendication 6, diverses concentrations des sondes et correspondant à différents intervalles de mesure de l'analyse et au moment actuel de la recherche.

10. Procédé selon la revendication 1, dans lequel les résultats de l'analyse à l'étape (d) (iii) consistant en une représentation graphique des profils des nucléotides terminaux du brin G d'un ADN télomérique humain sont utilisés, tels que la définition d'un critère dans le diagnostic médical de maladies et le vieillissement de l'organisme humain, comprenant une évaluation des propriétés prolifératives et des fonctions spécifiques aux tissus des cellules, la définition du pronostic de leur développement et un choix de la thérapie spécifique.

11. Jeu de désoxyribo-oligonucléotides spécifiques présentés dans SEQ ID NO: 1-5 pour l'amplification des brins négatifs de porte-à-faux G télomériques pendant une PCR dans un procédé selon la revendication 1.

12. Jeu de désoxyribo-oligonucleotides spécifiques selon la revendication 11 comprenant une quelconque séquence oligonucléotidique non complémentaire à des répétitions du brin G d'un ADN télomérique humain à la place de la séquence d'adaptateur présentée dans SEQ ID NO: 1-3.

13. Jeu de désoxyribo-oligonucléotides spécifiques présentés dans SEQ ID NO: 6-11 pour le marquage fluorescent de sondes signal lors d'une analyse duplex-spécifique dans un procédé selon la revendication 1.

**14.** Jeu de désoxyribo-oligonucléotides spécifiques selon la revendication 13, comprenant les différents types de marquage par une sonde fluorescente avec une combinaison « colorant-désactivateur » sur les extrémités opposées de la sonde consistant en de quelconques colorants fluorescents, comprenant le FITC, FAM, R110, TET, R6G, HEX, JOE, VIC, Yakima Yellow, TAMRA, Cy3, ROX, Super ROX, Cy 3.5, Cy5, Cy 5.5, Cy7, Cy 7.5, les désactivateurs RTQ, BHQ, le Dabcyl et d'autres.

Figure 1. Results of signal probe cleavage at the DSNP-analysis for equimolar mixture of the synthetic analogues of telomeric overhang nucleotide endings

Figure 2. Terminal Nucleotide Profile of the G-strand of human telomeric DNA, extracted from the culture of peripheral blood human lymphocytes, not stimulated to mitotic division by phytohemagglutinin

Figure 3. Terminal Nucleotide Profile of the G-strand of human telomeric DNA,
extracted from the culture of peripheral blood human lymphocytes, stimulated to
mitotic division by phytohemagglutinin during 2 hours

Figure 4. Terminal Nucleotide Profile of the G-strand of human telomeric DNA,
extracted from the culture of peripheral blood human lymphocytes, stimulated to
mitotic division by phytohemagglutinin during 4 hours

Figure 5. Terminal Nucleotide Profile of the G-strand of human telomeric DNA, extracted from the culture of peripheral blood human lymphocytes, stimulated to mitotic division by phytohemagglutinin during 11 hours

Figure 6. Terminal Nucleotide Profile of the G-strand of telomeric DNA for equimolar mixture of synthetic analogues of telomeric overhang nucleotide endings

Figure 7. Combined curves for the dynamics of changes of Terminal Nucleotide Profile on a time scale of PHA influence on the culture of human lymphocytes during first eleven hours

**Figure 8. Terminal Nucleotide Profile of the G-strand of human telomeric DNA for the leukemic culture Jurkat synchronized in the mitotic phase of cell cycle**

**Figure 9. Terminal Nucleotide Profile of the G-strand of human telomeric DNA for the leukemic culture K-562 synchronized in the mitotic phase of cell cycle**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- RU 201214326310

### Non-patent literature cited in the description

- **NADUPARAMBIL K. JACOB ; ROSE SKOPP ; CAROLYN M. PRICE.** G-overhang dynamics at Tetrahymena telomeres. *The EMBO Journal,* 2001, vol. 20 (15), 4299-4308
- **AGNEL J. SFEIR ; WEIHANG CHAI ; JERRY W. SHAY ; WOODRING E. WRIGHT.** Telomere-End Processing: the Terminal Nucleotides of Human Chromosomes. *Molecular Cell,* 2005, vol. 18, 131-138
- **YONG ZHAO ; AGNEL J. SFEIR et al.** Telomere extension occurs at most chromosome ends and is uncoupled from fill-in in human cancer cells. *Cell. Vol.,* 2009, vol. 138 (3), 463-475

- **SHAGIN D.A. et al.** A Novel Method for SNP Detection Using a New Duplex-Specific Nuclease from Crab Hepatopancreas. *Genome Research,* 2002, vol. 12, 1935-1942
- **YONG ZHAO et al.** Quantitative telomeric overhang determination using a double-strand specific nuclease. *Nucleic Acids Research,* 2008, vol. 36 (3
- **WOODRING E. WRIGHT et al.** Normal human chromosomes have long G-rich telomeric overhangs at one end. *Genes & Development,* 1997, vol. 11 (21), 2801-2809
- **ANISIMOVA V.E. et al.** Isolation, characterisation and molecular cloning of Duplex-Specific Nuclease from the hepatopancreas of Kamchatka crab. *BMC Biochemistry,* 2008, vol. 9 (14